(19) Europäisches Patentamt
European Patent Office
Office européen des brevets

(11) EP 3 049 809 B1

(12) FASCICULE DE BREVET EUROPEEN

(45) Date de publication et mention de la délivrance du brevet:
**04.07.2018 Bulletin 2018/27**

(21) Numéro de dépôt: **14771936.3**

(22) Date de dépôt: **24.09.2014**

(51) Int Cl.:
***G01N 33/50*** (2006.01) ***G01N 33/68*** (2006.01)

(86) Numéro de dépôt international:
**PCT/EP2014/070411**

(87) Numéro de publication internationale:
**WO 2015/044230 (02.04.2015 Gazette 2015/13)**

# (54) PROCÉDÉS D'ÉVALUATION DES EFFETS DÉLÉTÈRES DES UV SUR LA PEAU D'ENFANT

VERFAHREN ZUR BEWERTUNG DER SCHÄDLICHEN WIRKUNGEN VON UV-STRAHLUNG AUF DIE HAUT VON KINDERN

METHOD FOR EVALUATING THE HARMFUL EFFECTS OF UV ON CHILDREN'S SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.09.2013 FR 1359189**

(43) Date de publication de la demande:
**03.08.2016 Bulletin 2016/31**

(73) Titulaire: **Laboratoires Expanscience**
**92048 Paris La Défense Cedex (FR)**

(72) Inventeurs:
- **BAUDOUIN, Caroline**
  **78120 Rambouillet (FR)**
- **BRÉDIF, Stéphanie**
  **28210 Croisilles (FR)**
- **MSIKA, Philippe**
  **78000 Versailles (FR)**

(74) Mandataire: **Regimbeau**
**20, rue de Chazelles**
**75847 Paris Cedex 17 (FR)**

(56) Documents cités:
- **S Leclere-Bienfait ET AL: "Avocado perseose, a biomimetic active ingredient for the protection and accompaniment of infants' skin", Journal of investigative dermatology, 1 mai 2013 (2013-05-01), page S106, XP055095186, DOI: http://www.nature.com/jid/journal/v133/n1s /index.html#ab Extrait de l'Internet: URL:http://www.nature.com/jid/journal/v133 /n1s/pdf/jid201399a.pdf [extrait le 2014-01-08]**
- **DOMINIK PEUS ET AL: "Vitamin E analog modulates UVB-induced signaling pathway activation and enhances cell survival", FREE RADICAL BIOLOGY & MEDICINE, vol. 30, no. 4, 1 février 2001 (2001-02-01), pages 425-432, XP055123054, ISSN: 0891-5849, DOI: 10.1016/S0891-5849(00)00488-3**
- **WU ET AL: "IL-8 production and AP-1 transactivation induced by UVA in human keratinocytes: Roles of d-alpha-tocopherol", MOLECULAR IMMUNOLOGY, PERGAMON, GB, vol. 45, no. 8, 21 février 2008 (2008-02-21), pages 2288-2296, XP022513109, ISSN: 0161-5890, DOI: 10.1016/J.MOLIMM.2007.11.019**
- **C Baudouin et al: "Molecular biomarkers analysis to characterize epidermis of infants", Journal of investigative dermatology, 1 mai 2013 (2013-05-01), page S106, XP002725717, DOI: 10.1038/jid.2013.99 Extrait de l'Internet: URL:http://www.nature.com/jid/journal/v133 /n1s/pdf/jid201399a.pdf [extrait le 2014-01-08]**
- **ANGELA MARCHBANK ET AL: "The CUSP [Delta]Np63[alpha] isoform of human p63 is downregulated by solar-simulated ultraviolet radiation", JOURNAL OF DERMATOLOGICAL SCIENCE, vol. 32, no. 1, 1 juin 2003 (2003-06-01), pages 71-74, XP055123074, ISSN: 0923-1811, DOI: 10.1016/S0923-1811(03)00040-9**
- **K.M. LEE ET AL: "Analysis of genes responding to ultraviolet B irradiation of HaCaT keratinocytes using a cDNA microarray", BRITISH JOURNAL OF DERMATOLOGY, vol. 152, no. 1, 1 janvier 2005 (2005-01-01), pages 52-59, XP055123072, ISSN: 0007-0963, DOI: 10.1111/j.1365-2133.2005.06412.x**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 049 809 B1

- DAE-SUNG LEE ET AL: "Chronic ultraviolet radiation modulates epidermal differentiation as it up-regulates transglutaminase 1 and its substrates", PHOTODERMATOLOGY, PHOTOIMMUNOLOGY & PHOTOMEDICINE, vol. 21, no. 1, 1 février 2005 (2005-02-01), pages 45-52, XP055151477, ISSN: 0905-4383, DOI: 10.1111/j.1600-0781.2005.00131.x
- C D ENK ET AL: "The UVB-induced gene expression profile of human epidermis in vivo is different from that of cultured keratinocytes", ONCOGENE, vol. 25, no. 18, 27 avril 2006 (2006-04-27), pages 2601-2614, XP055151440, ISSN: 0950-9232, DOI: 10.1038/sj.onc.1209292
- A. S. PALLER ET AL: "New Insights About Infant and Toddler Skin: Implications for Sun Protection", PEDIATRICS, vol. 128, no. 1, 6 juin 2011 (2011-06-06), pages 92-102, XP055122877, ISSN: 0031-4005, DOI: 10.1542/peds.2010-1079

## Description

La présente invention concerne des procédés *in vitro* de test de formulations ou d'actifs pour la prévention des effets délétères des UV sur la peau d'enfant, en particulier âgé de trois ans ou moins.

La peau humaine subit quotidiennement l'exposition aux rayonnements ultraviolets (UV), qui, dans une certaine mesure sont bénéfiques pour la santé humaine.

Il est par exemple bien connu que l'exposition solaire permet la synthèse de vitamine D, dont le déficit entraine des troubles de la croissance. Par ailleurs, certaines dermatoses comme le psoriasis, la dermatite atopique et certains vitiligos, sont améliorés par les UV naturels ou artificiels qui induisent une diminution de ces lésions.

Une exposition excessive aux UV, qu'elle soit ponctuelle ou chronique, peut cependant avoir des conséquences pathologiques au niveau cutané.

Le coup de soleil est l'effet aigu le mieux connu d'une exposition excessive ponctuelle aux UV, en cas d'ensoleillement important par exemple.

Cette réaction des tissus résulte d'altérations cellulaires au site des lésions, dues aux effets délétères des UV. En effet, des intensités de rayonnement UV élevées endommagent les cellules de la couche superficielle de la peau.

Dans sa forme la plus bénigne, le coup de soleil consiste en un rougissement de la peau appelé érythème. Celui-ci survient peu après l'exposition aux UV et atteint son maximum d'intensité dans les 8 à 24 heures qui suivent. Il disparaît en quelques jours. Toutefois, un fort coup de soleil peut provoquer l'apparition de cloques ou faire "peler" la peau. La « nouvelle » peau ainsi mise à jour est dépourvue de la protection de la couche cornée, elle est donc particulièrement fragile. En outre, dans le cas de coups de soleil importants, les cloques sont susceptibles de laisser ultérieurement place à des cicatrices au site de la lésion.

A long terme, l'exposition excessive chronique aux UV provoque des lésions dégénératives dans les cellules cutanées, le tissu fibreux et les vaisseaux sanguins, conduisant à un vieillissement prématuré de la peau, à des photodermatoses et à des kératoses actiniques. Le cancer de la peau, le plus souvent sous la forme d'un mélanome malin, est la lésion dégénérative la plus connue résultant d'une exposition chronique aux UV trop importante.

La gravité et la fréquence de ces réactions cutanées ponctuelles ou chronique dépend non seulement de la quantité et du type d'UV auxquels la peau est exposée, mais aussi au type même de la peau. Il est bien connu en effet que le phototype de la peau joue un grand rôle dans la réaction aux UV, tant dans le risque de coup de soleil que dans le risque de développer un mélanome malin.

En outre, on considère généralement que la peau des enfants est particulièrement sensible aux UV, et ce d'autant plus que les enfants sont jeunes. On sait en effet que les enfants sont plus susceptibles que les adultes de développer des réactions de type « coups de soleil » après une exposition aux UV. Il semble par ailleurs que l'exposition au soleil pendant l'enfance conditionne l'apparition à l'âge adulte de cancers cutanés (Stanton WR, Health Promot Int.;19(3):369-78; 2004). En effet, les dommages cutanés induits par les UV pourraient s'accumuler dès le premier été de la vie (Paller AS et al, Pediatrics ;128 :92-10 ; 2011).

En effet, avant l'âge de 3 ans, les enfants n'ont presque pas de défenses face au soleil. Leur peau encore immature les rendrait ainsi particulièrement sensibles aux rayons solaires, laissant passer quantité d'UVA et UVB qui agressent les couches basales de l'épiderme. Dans des modèles animaux, il a été démontré que les UV induisent plus de mutations sur l'ADN des mélanocytes de souris nouveau nés comparativement aux souris adultes, ceci générant donc plus de tumeurs (Wolnicka-Glubisz A and Noonan FP, Photochem Photobiol Sci.; 5:254; 2006).

Il est donc généralement admis qu'il est nécessaire de protéger la peau des enfants, bien que la variété et l'étendue des effets des UV au niveau cellulaire et moléculaire dans cette peau spécifique ne soient pas connues avec précision. Faute de connaitre les spécificités de la réaction aux UV chez l'enfant, les produits développés pour l'adulte sont classiquement utilisés chez l'enfant.

Cependant, les inventeurs ont découvert que la peau des enfants très jeunes, c'est-à-dire âgés de trois ans ou moins, répond aux expositions aux UV de manière spécifique. Autrement dit, il n'est pas possible d'estimer quelle sera la réaction d'une peau très jeune exposée aux UV sur la base de ce qui est connu de la peau des adultes.

Ainsi, par exemple, les inventeurs ont montré que, suite à une exposition à des UVA et des UVB, le niveau d'expression de certains marqueurs augmente ou diminue dans la peau des enfants âgés de trois ans ou moins de façon très importante en comparaison avec la peau d'enfants moins jeunes. Ces modulations (augmentation ou diminution) ne sont pas homogènes pour l'ensemble des marqueurs connus pour être stimulés par les UV. Ainsi, le profil d'expression de la peau d'enfant âgé de trois ans ou moins ne peut aucunement être extrapolé simplement à partir de résultats relatifs à la peau d'enfant moins jeune, ou à la peau d'adulte.

Malheureusement, il n'existe à ce jour aucune méthode permettant d'évaluer l'efficacité de produits solaires sur la peau très spécifique des enfants très jeunes, âgés de trois ans ou moins. En conséquence, l'absence de procédés de tests *in vitro* adaptés entrave le développement de produits cosmétiques ou thérapeutiques sûrs et efficaces qui leur soient destinés.

Il existe donc bien un besoin pour des méthodes in vitro permettant d'évaluer l'efficacité de formulations ou

d'actifs dans la prévention des effets délétères des UV sur la peau des enfants âgés de trois ans ou moins.

Leclere-Bienfait et al. (2013, J. Invest. Dermatol., S106) décrit une méthode d'identification d'un actif (le perséose d'avocat) capable d'accompagner le développement de la peau de nourrissons tout en étant bien toléré. Leclere-Bienfait et al. indique que le traitement de la peau par cet actif permet de limiter l'effet induit par l'irradiation aux UV sur les niveaux d'expression de deux marqueurs particuliers, les intégrines alpha 6 et beta 1 dans des kératinocytes isolés d'échantillons de peau de bébé et d'enfant.

Peus et al. (2001, Free Rad Biol & Med, 30(4), 452-432) décrit l'effet d'un actif sur les niveaux d'activation de protéines du cycle et de la prolifération cellulaire dans une culture de kératinocytes issus d'échantillons de peau de nouveau-né et soumise à un rayonnement UVB.

Wu et al. (2008, Mol Immunol, 45(8), 2288-2296) décrit l'effet d'un actif sur le niveau d'expression de l'interleukine IL8 et sur l'activité du facteur de transcription AP-1, dans une culture de kératinocytes issus d'échantillons de peau de nouveau-né et soumise à un rayonnement UVA.

Baudouin et al. (2013, J. Invest. Dermatol., S106) décrit l'analyse globale de l'expression des gènes par des kératinocytes isolés à partir de différents échantillons de peau de bébé et d'enfant.

Marchbank et al. (2003, J Derm Sci, 32, 71-74) décrit l'effet de l'irradiation par rayonnement UVA et UVB sur le niveau d'expression d'un isoforme de la protéine p63 dans une culture de kératinocytes issus d'échantillons de peau de nouveau-né.

Lee et al. (2005, Brit J Dermatol, 152, 52-59) décrit l'analyse globale de l'expression des gènes chez des kératinocytes de la lignée cellulaire HaCat exposés au rayonnement UVB.

Lee et al. (2005, Photodermatology, Photoimmunology & Photomedecine, 21, 45-52) décrit l'effet de l'irradiation aux UV sur la peau de souris et sur des échantillons de peau humaine provenant d'individus adultes âgés de 65 ans ou plus.

Enk et al. (2006, Oncogene, 25, 2601-2614) décrit l'analyse globale des niveaux d'expression des gènes dans des échantillons de peau d'individus humains ou dans des cultures de kératinocytes d'origine humaine après exposition aux UVB.

**Légendes des figures**

Figure 1 : analyse histologique et de la formation des sunburn cells dans les épidermes reconstruits irradiés. ↘ Sunburn (SB) cell (A) épiderme reconstruit 3 mois non irradié ; (B) épiderme reconstruit 3 mois irradié UVA+B - 574 SB Cell/mm$^2$ ; (C) épiderme reconstruit 11 ans non irradié ; (D) épiderme reconstruit 11 ans irradié UVA+B - 529 SB Cell/mm$^2$.

Figure 2 : Profil des gènes de l'inflammation induits par les UV - Comparaison des épidermes 3 mois et 11 ans.

Figure 3 : Profil des gènes de la défense antioxydante, antimicrobienne et de protection de l'ADN réprimés par les UV - Comparaison des épidermes 3 mois et 11 ans.

Figure 4 : Profil des gènes de la différenciation épidermique et de la fonction barrière réprimés par les UV - Comparaison des épidermes 3 mois et 11 ans.

Figure 5 : Profil des gènes des interactions cellule-cellule, de l'adhésion à la matrice et de la réparation épidermique réprimés par les UV - Comparaison des épidermes 3 mois et 11 ans.

Figure 6 : Profil des gènes des cellules souches réprimés par les UV - Comparaison des épidermes 3 mois et 11 ans.

Figure 7 : Immunomarquage de l'intégrine alpha 6 dans des épidermes reconstruits irradiés. (A) épiderme reconstruit 3 mois non irradié ; (B) épiderme reconstruit 3 mois irradié UVA+B; (C) épiderme reconstruit 11 ans non irradié ;

(D) épiderme reconstruit 11 ans irradié UVA+B.

Figure 8 : analyse histologique et de la formation des sunburn cells dans les épidermes reconstruits de 3 mois irradiés sans ou en présence d'une crème solaire contenant du perséose d'avocat. ↘ Sunburn (SB) cells ; (A) épiderme reconstruit 3 mois irradié UVA+B - 574 SB Cell/mm$^2$, (B) épiderme reconstruit 3 mois irradié UVA+B en présence d'une crème solaire - 0 SB cell/mm$^2$.

**Description détaillée**

Les inventeurs ont montré que la peau des enfants très jeunes, c'est-à-dire les enfants âgés de trois ans ou moins, préférablement de deux ans ou moins, plus préférablement d'un an ou moins, encore plus préférablement de six mois ou moins, et en particulier de trois mois ou moins, est particulièrement sensible aux effets délétères des UV. Ils ont en particulier montré que la peau des enfants de trois ans ou moins réagit à l'exposition aux UVA et/ou aux UVB d'une manière distincte non seulement de la peau des adultes, mais aussi de la peau d'enfants âgés d'environ 11 ans. En particulier, les inventeurs ont mis en évidence la plus grande importance des effets délétères des UV sur les épidermes de 3 mois, montrant ainsi la plus grande sensibilité au soleil de la peau des nourrissons et des jeunes enfants.

Les inventeurs ont développé des procédés d'évaluation de l'efficacité *in vitro* de formulations sur la prévention des effets délétères des UV pour la peau d'enfant âgé de trois ans ou moins, utilisant un modèle de peau spécifiquement capable de reproduire les caractéristiques de la peau des enfants de cet âge. Les procédés de l'invention reposent donc sur l'utilisation d'un modèle de peau adapté, reproduisant la peau d'enfant âgé de trois ans ou moins, ainsi que sur l'utilisation de marqueurs biologiques, dont le niveau d'expression est affecté par les UV de manière particulière dans la peau des enfants de trois ans ou moins, préférablement de deux ans ou moins, plus préférablement d'un an ou moins, encore plus préférablement de six mois ou moins, et en particulier de trois mois ou moins, en comparaison avec la peau d'enfants plus âgés ou d'adultes.

Les procédés de l'invention sont aussi adaptés à l'évaluation de l'activité de principes actifs. L'invention permet ainsi de déterminer de façon précise quels actifs ont un effet avantageux sur la prévention ou le traitement des effets délétères dus aux UV sur la peau.

Il est ici décrit un procédé d'évaluation de l'efficacité *in vitro* d'une formulation ou d'un actif sur la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins, caractérisé en ce que ledit procédé comprend les étapes suivantes:

a) mettre en contact ladite formulation ou ledit actif avec un modèle de peau comprenant des kératinocytes obtenus à partir de sujets âgés de 3 ans ou moins, préférablement de deux ans ou moins, plus préférablement d'un an ou moins, encore plus préférablement de six mois ou moins, et en particulier de trois mois ou moins;
b) exposer le modèle de peau de l'étape a) aux UV ;
c) mesurer le niveau d'expression d'au moins un marqueur biologique dans le modèle de peau de l'étape b), caractérisé en ce que ledit marqueur biologique est choisi parmi les marqueurs de l'inflammation cutanée ;
d) comparer le niveau d'expression d'au moins un marqueur biologique obtenu à l'étape c) avec un niveau d'expression de référence ;
e) évaluer l'efficacité de ladite formulation ou dudit actif sur la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins, en fonction de la comparaison de l'étape d).

Par « l'efficacité d'une formulation ou d'un actif dans la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins », on entend ici la capacité de la formulation ou de l'actif à annuler ou diminuer lesdits effets délétères sur la peau d'enfant âgé de trois ans ou moins, préférablement de deux ans ou moins, plus préférablement d'un an ou moins, encore plus préférablement de six mois ou moins, et en particulier de trois mois ou moins.

L'invention concerne en particulier un procédé tel que revendiqué dans la revendication 1.

L'invention permet d'isoler des formulations ou des actifs ayant un effet dans la prévention des effets délétères des UV sur la peau d'enfant âgé de trois ans ou moins. L'invention permet donc d'identifier des formulations ou des actifs appropriés à cette peau très spécifique.

Il est donc également ici décrit un procédé d'identification d'une formulation ou d'un actif pour la prévention des effets délétères des UV sur la peau d'enfant âgé de trois ans ou moins, caractérisé en ce que ledit procédé comprend les étapes suivantes:

a) mettre en contact une formulation ou un actif candidat avec un modèle de peau comprenant des kératinocytes obtenus à partir de sujets âgés de 3 ans ou moins ;

b) exposer le modèle de peau de l'étape a) aux UV ;

c) mesurer le niveau d'expression d'au moins un marqueur biologique dans le modèle de peau de l'étape b), caractérisé en ce que ledit marqueur biologique est choisi parmi les marqueurs de l'inflammation cutanée.

d) comparer le niveau d'expression d'au moins un marqueur biologique obtenu à l'étape c) avec un niveau d'expression de référence ;

e) déterminer si ladite formulation ou ledit actif candidat est une formulation ou un actif pour la prévention des effets délétères des UV sur la peau d'enfant âgé de trois ans ou moins en fonction de la comparaison de l'étape d).

L'invention concerne en particulier un procédé tel que revendiqué dans la revendication 2.

La formulation candidate est une formulation pour la prévention des effets délétères des UV sur la peau d'enfant âgé de trois ans ou moins, si ladite formulation candidate permet de moduler l'expression d'au moins un marqueur biologique du procédé. Cette modulation peut correspondre, selon les cas, et en particulier selon la nature du marqueur biologique, à une augmentation ou à une diminution de l'expression dudit marqueur. Par exemple, il peut être intéressant d'isoler des formulations minimisant les effets délétères des UV sur les marqueurs préférentiellement exprimés dans les cellules souches, ces formulations permettant de préserver la capacité de renouvellement de l'épiderme fragile des enfants de trois ans ou moins.

De la même façon, l'actif candidat est un actif pour la prévention des effets délétères des UV sur la peau d'enfant âgé de trois ans ou moins, si ledit actif candidat permet de moduler l'expression d'au moins un marqueur biologique du procédé. Cette modulation peut correspondre, selon les cas, et en particulier selon la nature du marqueur biologique, à une augmentation ou à une diminution de l'expression dudit marqueur.

Par « effets délétères des UV», on entend ici les réactions pathologiques découlant de l'exposition de la peau aux UV, c'est-à-dire aux UVA et/ou aux UVB. Ces réactions pathologiques comprennent notamment l'érythème, la formation de cellules dites « sunburn cells », l'altération de la barrière et de l'épiderme, la formation de lésions, la desquamation, l'altération de la physiologie des cellules souches et les dommages à l'ADN des cellules irradiées. Comme cela est bien connu de l'homme du métier, les « sunburn cells » sont des kératinocytes ayant subi les effets délétères des UV. Les sunburn cells sont des kératinocytes apoptotiques qui peuvent facilement être identifié par analyse histochimique, de par leur morphologie caractéristique, à savoir un noyau basophile sombre, pycnotique et condensé, un cytoplasme éosiphile et la formation d'espaces intercellulaires.

Au sens du procédé, l'expression « l'efficacité d'une formulation dans la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins » signifie la capacité de la formulation à diminuer au moins l'un des effets délétères susmentionnés.

Dans un premier temps, la formulation d'intérêt est mise en contact avec un modèle de peau comprenant des kératinocytes obtenus à partir de sujets âgés de 3 ans ou moins, préférablement de deux ans ou moins, plus préférablement d'un an ou moins, encore plus préférablement de six mois ou moins, et en particulier de trois mois ou moins.

De la même façon, la mise en contact de l'actif d'intérêt avec le modèle de peau peut être faite directement, si la formulation de l'actif le permet. Dans certains cas, il pourra être avantageux de formuler l'actif d'intérêt, par exemple de façon à obtenir une composition liquide, afin de faciliter sa mise en contact avec le modèle de peau. Ainsi, par exemple, le procédé comprend en outre une étape de formulation de l'actif, notamment sous forme d'une solution liquide, en particulier aqueuse, préalablement à l'étape de mise en contact dudit actif avec un modèle de peau.

Au sens du procédé, le modèle de peau comprenant des kératinocytes obtenus à partir de sujets âgés de 3 ans ou moins, préférablement de deux ans ou moins, plus préférablement d'un an ou moins, encore plus préférablement de six mois ou moins, et en particulier de trois mois ou moins, peut être tout modèle tissulaire comprenant des kératinocytes et dans lequel les kératinocytes ont été obtenus à partir de sujets âgés de 3 ans ou moins, préférablement de deux ans ou moins, plus préférablement d'un an ou moins, encore plus préférablement de six mois ou moins, et en particulier de trois mois ou moins.

Les inventeurs ont précédemment montré que les profils d'expression de catégories spécifiques de gènes (par exemple, gènes de la barrière, de l'inflammation, de défense, des cellules souches) évolue en fonction de l'âge (demande PCT/EP2013/064926). L'homme du métier peut ainsi aisément caractériser la peau au niveau moléculaire depuis la naissance jusqu'à l'âge adulte. Plus particulièrement l'homme du métier notera que les cellules de la peau d'enfant de trois ans ou moins, dont les kératinocytes, présentent un profil d'expression spécifique des gènes impliqués dans des processus physiologiques particuliers, notamment le métabolisme cellulaire, la réponse au stress, l'inflammation, l'immunité, l'apoptose, la croissance/prolifération et le cycle cellulaire, la signalisation cellulaire, la migration et la différenciation, la barrière épidermique, l'adhésion et les cellules souches pluripotentes de la peau.

Ainsi, le modèle de peau du procédé peut être tout modèle tissulaire comprenant des kératinocytes obtenus à partir de sujets âgés de 3 ans ou moins, préférablement de deux ans ou moins, plus préférablement d'un an ou moins,

encore plus préférablement de six mois ou moins, et en particulier de trois mois ou moins. Le modèle de peau du procédén'est donc pas strictement limité à un type de modèle tissulaire particulier, et peut être adapté aux besoins de l'homme du métier.

Par « modèle tissulaire comprenant des kératinocytes », on entend au sens du procédé toute culture *in vitro* de cellules cutanées comprenant au moins des kératinocytes. Ainsi, les « modèles tissulaires comprenant des kératinocytes » selon le procédé comprennent les cultures de kératinocytes en monocouche, les cultures de cellules cutanées en bicouche comprenant des kératinocytes, ainsi que les modèles tissulaires tels que les cultures de peaux reconstruites comprenant des kératinocytes. Avantageusement, le modèle de peau comprend les cultures de peau reconstruite comprenant des kératinocytes.

Par exemple, les cellules cutanées comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon le procédécomprennent au moins des kératinocytes et/ou des fibroblastes.

Les cultures de cellules cutanées en monocouche ou en bicouche sont connues et utilisées depuis très longtemps, et ne nécessitent pas de description détaillée.

Par ailleurs, de nombreux modèles de peaux reconstruites sont à la disposition de l'homme du métier (qui pourra se référer en particulier à Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000 ; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009 ; Kinicoglu et al., Biomaterials, 32(25) : 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1) : 47-55, 2012 ; EP 29 678 ; EP 285 471 ; EP 789 074 ; EP 1 451 302 B1 : EP 1 878 790 B1 ; EP 1 974 718 ; US 2007/0148,771 ; US 2010/0,099,576 ; WO 02/070729 ; WO 2006/063864 ; WO 2006/0,63865 ; WO 2007/064305).

Préférablement, le modèle de peau reconstruite est sélectionné dans le groupe comprenant les modèles d'épiderme constitués principalement de kératinocytes, les modèles de peau comprenant un derme et un épiderme, et les modèles de peau comprenant un hypoderme, un derme, et un épiderme. Les modèles comprenant au moins un épiderme forment des épithélia stratifiés comprenant les couches caractéristiques du tissu considéré. Par exemple, on peut identifier dans les modèles d'épiderme une couche basale (stratum basalis), une couche épineuse (stratum spinosum), une couche granuleuse (stratum granulosum), et une couche cornée (stratum corneum).

Avantageusement, le modèle de peau du procédé est un modèle d'épiderme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable.
- Dans ce groupe on trouve les modèles Epiderme reconstruits (Skinethic®) ainsi que le modèle EpiDerm®, (Mattek Corporation) ;
- un film, une membrane ou une matrice à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin ® (L'Oréal).

Ces modèles peuvent en outre être ensemencés ou non par des fibroblastes dans la partie dermique.

Avantageusement, sont introduites dans le modèle de peau du procédé, des cellules pigmentaires, des cellules immunocompétentes, des cellules nerveuses, de préférence les cellules immunocompétentes sont des cellules de Langerhans. De façon générale, lorsque le modèle de peau du procédé est un modèle d'épiderme, le support matriciel est produit et ensuite ensemencé par les kératinocytes pour reconstruire l'épiderme et obtenir finalement une peau reconstruite comprenant des kératinocytes.

Dans le contexte du procédé, le terme « kératinocytes » désigne à la fois des kératinocytes primaires et des kératinocytes immortalisés, comme par exemple des kératinocytes provenant de lignées cellulaires.

Par exemple, les kératinocytes, primaires ou immortalisés, sont d'origine humaine. Par exemple, les kératinocytes proviennent d'échantillons biologiques provenant d'un sujet humain âgés de 3 ans ou moins, préférablement de

deux ans ou moins, plus préférablement d'un an ou moins, encore plus préférablement de six mois ou moins, et en particulier de trois mois ou moins. Il va de soi cependant que par l'expression « obtenus à partir de sujets âgés de 3 ans ou moins, préférablement de deux ans ou moins, plus préférablement d'un an ou moins, encore plus préférablement de six mois ou moins, et en particulier de trois mois ou moins », le présent procédé ne fait pas référence à des kératinocytes prélevés *in utero*, ou à partir de sujets non encore nés. Le présent procédé exclut donc clairement l'utilisation de cellules embryonnaires.

Les kératinocytes humains peuvent en particulier être isolés à partir d'échantillons de peau, par des techniques de culture cellulaire bien connues de l'homme du métier.

Par exemple, les kératinocytes peuvent être des kératinocytes obtenus à partir d'explant de tissu cutané, en particulier des échantillons d'épithélium malpighien kératinisé. Les kératinocytes humains peuvent ensuite facilement être cultivés *in vitro* selon des techniques bien connues. L'homme du métier pourra en particulier se référer à Leigh et al. (Arch Dermatol Res.;286(1):53-61.1994).

Par « explant » ou « explant de peau », on entend ici un prélèvement de cellules ou de tissu cutané, lequel peut être réalisé dans un but thérapeutique ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède par exemple à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse au sens du procédé inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés dans le présent procédé.

Par « kératinocytes immortalisés » on entend au sens de la présente demande des kératinocytes qui se divisent au-delà de la limite de Hayflick. Ces cellules ont ainsi acquis la capacité de se multiplier indéfiniment, soit suite à une mutation aléatoire ou à une modification délibérée.

Il est bien connu que les cellules normales ne peuvent se diviser qu'un nombre déterminé de fois. Une fois cette limite atteinte, les cellules deviennent sénescentes puis meurent. La limite de Hayflick correspond au nombre de divisions qui peuvent être effectuées par une cellule normale, c'est-à-dire une cellule ne pouvant se diviser qu'un nombre déterminé de fois, avant de cesser de se diviser. Au sens du procédé, la limite de Hayflick correspond au nombre de divisions qui peuvent être effectuées par un kératinocyte normal avant de cesser de se diviser.

Les kératinocytes peuvent être immortalisés suite à des anomalies particulières, comme c'est le cas par exemple des cellules cancéreuses, ou suite à la mise en oeuvre d'une technique d'immortalisation. Les techniques d'immortalisation sont bien connues de l'homme de l'art qui peut facilement choisir parmi celles-ci la technique la mieux adaptée à son objectif. Par exemple, et sans limiter le procédé à ces exemples, on peut citer comme technique d'immortalisation la transformation par un oncogène, en particulier par l'antigène T de SV40, la protéine Ras, la protéine myc, la protéine Abl. Comme autres techniques on peut citer par exemple la surexpression d'une transcriptase inverse de la télomérase, par exemple hTERT, ou la culture des kératinocytes à confluence de plusieurs passages. Toutes ces techniques sont des techniques de biologie cellulaire classiques qui n'ont pas besoin d'être détaillées ici.

Après avoir mis en contact la formulation d'intérêt et le modèle de peau du procédé, l'homme du métier pourra exposer le modèle de peau ainsi traité aux UV.

Par « exposer le modèle de peau aux UV » on entend ici toute exposition à un rayonnement comprenant ou consistant en des rayons ultraviolets.

Au sens du procédé, les termes « rayons ultraviolets » se réfèrent à des rayons électromagnétiques dont les longueurs d'ondes sont comprises entre 153 nm et 400 nm. Préférentiellement, l'exposition aux UV selon le procédé comprend l'exposition à des UVA et/ou à des UVB. Par UVA, on entend ici des rayonnements dont la longueur d'onde est comprise entre 400 et 315 nm. Par UVB, on entend ici des rayonnements dont la longueur d'onde est comprise entre 315 et 280 nm.

Ainsi, de préférence, l'exposition aux UV selon le procédé comprend ou consiste en l'exposition à des UVA. De préférence encore, l'exposition aux UV selon le procédé comprend ou consiste en l'exposition à des UVB. Avantageusement, l'exposition aux UV selon le procédé comprend ou consiste en l'exposition à des UVA et à des UVB.

L'exposition d'un modèle *in vitro* aux UV est une technique de routine bien connue de l'homme de l'art. Au sens du procédé, cette exposition peut être réalisée selon toute modalité choisie par l'homme de l'art. Celui-ci pourra

en particulier se référer à Diffey BL et al. (J Am Acad Dermatol; 43(6):1024-35; 2000), pour ce qui est des méthodes standard de références d'exposition aux UV utilisées dans le domaine cosmétique.

Après avoir exposé le modèle de peau du procédé aux UV, l'homme du métier pourra procéder à la mesure du niveau d'expression des marqueurs biologiques du procédé.

Par « marqueur biologique », on entend ici une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances et de paramètre divers. Par exemple, un marqueur biologique peut être une substance dont la détection indique un état pathologique particulier (par exemple la présence de la protéine C activée en tant que marqueur d'une infection), ou au contraire une substance dont la détection indique un état physiologique spécifique. Le marqueur biologique du procédéest préférentiellement un gène, les produits d'un gène tels que ses transcrits et les peptides issus de ses transcrits, un lipide, un sucre ou un métabolite.

Par exemple, le marqueur biologique est un gène, les produits d'un gène tels que des transcrits ou des peptides, un lipide, un sucre ou un métabolite dont les changements d'expression, en particulier de niveau d'expression, corrèlent avec un état physiologique de la peau d'enfant âgé de trois ans ou moins, préférablement de deux ans ou moins, plus préférablement d'un an ou moins, encore plus préférablement de six mois ou moins, et en particulier de trois mois ou moins. En particulier, le marqueur biologique est un peptide ayant une activité enzymatique.

Les inventeurs ont mis en évidence que les marqueurs de l'inflammation cutanée peuvent être utilisés pour observer la réaction de la peau d'enfant âgé de trois ans ou moins, préférablement de deux ans ou moins, plus préférablement d'un an ou moins, encore plus préférablement de six mois ou moins, et en particulier de trois mois ou moins, après une exposition aux UV.

Par « marqueurs de l'inflammation cutanée », on entend ici les marqueurs dont la variation d'expression corrèle avec l'inflammation cutanée.

L'inflammation cutanée est une réaction du système immunitaire bien connue de l'homme de l'art, qui peut se manifester par un érythème, caractérisé par une rougeur associée à une vasodilatation locale, un oedème, caractérisée par un gonflement, et une sensation de chaleur. En outre, l'inflammation cutanée s'accompagne d'une variation de niveau d'expression ou de concentration de marqueur géniques ou protéiques bien connus de l'homme du métier, qui pourra se référer par exemple à Vahlquist (Acta Derm Venereol; 80: 161 ; 2000).

L'homme du métier cherchant à déterminer à quelle classe appartient un marqueur génique ou protéique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Les inventeurs ont particulièrement sélectionné des marqueurs dont la variation du niveau d'expression varie après exposition aux UV de manière surprenante et inattendue chez les enfants âgés de trois ans ou moins, préférablement de deux ans ou moins, plus préférablement d'un an ou moins, encore plus préférablement de six mois ou moins, et en particulier de trois mois ou moins. En effet, le profil d'expression de ces marqueurs est particulièrement intéressant en ce qu'il diffère grandement et de façon non prévisible du profil d'expression observé dans des modèles de peau d'adulte, ou d'enfant âgé d'environ 11 ans.

Les marqueurs sélectionnés présentent donc un intérêt particulier dans le cadre de la méthode, dans la mesure où leur niveau d'expression est mesuré sur un modèle de peau reproduisant les caractéristiques de la peau d'enfant âgé de trois ans ou moins.

Selon le procédé, le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3.

Au sens du présent procédé, le marqueur biologique PTGS2 comprend le gène PTGS2 humain (référence NCBI : Gene ID: 5743), ainsi que les produits de ce gène. En particulier, le marqueur biologique PTGS2 consiste en le gène PTGS2 humain (référence NCBI : Gene ID: 5743). Par exemple, le marqueur biologique PTGS2 consiste en l'un des produits du gène PTGS2 humain. Les produits du gène PTGS2 humain comprennent le transcrit du gène PTGS2 humain et la protéine humaine Synthase prostaglandine-endoperoxyde 2, également connue sous le nom de cyclooxygénase-2 ou de COX-2. Au sens du présent procédé, le transcrit du gène PTGS2 humain est le polynucléotide dont la séquence a pour référence NCBI : NM_000963. Par « COX-2 humaine », on entend ici la protéine dont la séquence peptidique est la séquence de référence NCBI : NP_000954.

Au sens du présent procédé, le marqueur biologique IL1α comprend le gène IL1A humain (référence NCBI : Gene ID: 3552), ainsi que les produits de ce gène. En particulier, le marqueur biologique IL1α consiste en le gène IL1A humain (référence NCBI : Gene ID: 3552). Par exemple, le marqueur biologique IL1A consiste dans l'un des produits du gène IL1A humain. Les produits du gène IL1A humain comprennent le transcrit du gène IL1A humain et la protéine humaine IL1α. Au sens du présent procédé, le transcrit du gène IL1A humain est le polynucléotide dont la séquence a pour référence NCBI : NM_000575. Par « protéine humaine IL1α », on entend ici la protéine dont la séquence peptidique

est la séquence de référence NCBI : NP_000566

Au sens du présent procédé, le marqueur biologique IL8 comprend le gène IL8 humain (référence NCBI : Gene ID: 3576), ainsi que les produits de ce gène. En particulier, le marqueur biologique IL8 consiste dans le gène IL8 humain (référence NCBI : Gene ID: 3576). Par exemple, le marqueur biologique IL8 consiste en l'un des produits du gène IL8 humain. Les produits du gène IL8 humain comprennent le transcrit du gène IL8 humain et la protéine humaine IL8. Au sens du présent procédé, le « transcrit du gène IL8 humain » est le polynucléotide dont la séquence a pour référence NCBI : NM_000584. Par « protéine humaine IL8 », on entend ici la protéine dont la séquence peptidique est la séquence de référence NCBI : NP_000575.

Au sens du présent procédé, le marqueur biologique IL1-R1 comprend le gène IL1-R1 humain (référence NCBI : Gene ID: 3554), ainsi que les produits de ce gène. En particulier, le marqueur biologique IL1-R1 consiste en le gène IL1-R1 humain (référence NCBI : Gene ID: 3554). Par exemple, le marqueur biologique IL1-R1 consiste en l'un des produits du gène IL1-R1 humain. Les produits du gène IL1-R1 humain comprennent le transcrit du gène IL1-R1 humain et le précurseur à la protéine IL1-R1 humaine. Au sens du présent procédé, le transcrit du gène IL1-R1 humain est le polynucléotide dont la séquence a pour référence NCBI : NM_000877.2. Par « le précurseur à la protéine IL1-R1 humaine », on entend ici la protéine dont la séquence peptidique est la séquence de référence NCBI : NP_000868.1.

Au sens du présent procédé, le marqueur biologique IL1-RN comprend le gène IL1RN humain (référence NCBI : Gene ID : 3557), ainsi que les produits de ce gène. En particulier, le marqueur biologique IL1-RN consiste en le gène IL1-RN humain (référence NCBI : Gene ID : 3557). Par exemple, le marqueur biologique IL1-RN consiste en l'un des produits du gène IL1-RN humain. Les produits du gène IL1-RN humain comprennent le transcrit du gène IL1-RN humain et la protéine humaine IL-1RA. Au sens du présent procédé, le transcrit du gène IL1-RN humain est le polynucléotide dont la séquence a pour référence NCBI : NM_000577. Par « protéine humaine IL-1RA humaine », on entend ici la protéine dont la séquence peptidique est la séquence de référence NCBI : NP_000568.1.

Au sens du présent procédé, le marqueur biologique MMP1 comprend le gène MMP1 humain (référence NCBI : Gene ID: 3557), ainsi que les produits de ce gène. En particulier, le marqueur biologique MMP1 consiste en le gène MMP1 humain (référence NCBI : Gene ID: 4312). Par exemple, le marqueur biologique MMP1 consiste en l'un des produits du gène MMP1 humain. Les produits du gène MMP1 humain comprennent le transcrit du gène MMP1 humain et la protéine MMP1 humaine. Au sens du présent procédé, le transcrit du gène MMP1 humain est le polynucléotide dont la séquence a pour référence NCBI : NM_001145938. Par « protéine MMP1 humaine », on entend ici la protéine dont la séquence peptidique est la séquence de référence NCBI : NP_001139410.

Au sens du présent procédé, le marqueur biologique MMP3 comprend le gène MMP3 humain (référence NCBI : Gene ID: 4314), ainsi que les produits de ce gène. En particulier, le marqueur biologique MMP3 consiste en le gène MMP3 humain (référence NCBI : Gene ID: 4314). Par exemple, le marqueur biologique MMP3 consiste en l'un des produits du gène MMP3 humain. Les produits du gène MMP3 humain comprennent le transcrit du gène MMP3 humain et la protéine MMP3 humaine. Au sens du présent procédé, le transcrit du gène MMP3 humain est le polynucléotide dont la séquence a pour référence NCBI : NM_002422. Par « protéine humaine MMP3 humaine », on entend ici la protéine dont la séquence peptidique est la séquence de référence NCBI : NP_002413.

En outre, l'homme du métier comprendra qu'il est possible dans le cadre du procédé de mesurer d'autres marqueurs biologiques pertinents. L'utilisation de marqueurs biologiques supplémentaires peut ainsi permettre une évaluation de l'efficacité des formulations ou actifs d'intérêt plus complète. Au sens du procédé, des marqueurs biologiques pertinents sont par exemple être des marqueurs biologiques connus pour être exprimés dans la peau suite à l'exposition aux UV.

En particulier, comme détaillé dans la partie expérimentale, les inventeurs ont montré que les marqueurs de la défense antioxydante antimicrobienne ou de l'ADN, les marqueurs de la barrière épidermique, les marqueurs de l'adhésion et de la réparation cutanée et les marqueurs préférentiellement exprimés dans les cellules souches étaient exprimés de façon différente dans les modèles de peau de bébé exposés aux UV, et dans les modèles de peau d'enfants âgés d'environ 11 ans, traités dans les mêmes conditions.

Ces marqueurs peuvent donc avantageusement être utilisés dans les procédés ici décrits.

Ainsi, de préférence, le procédé est caractérisé en ce que l'étape c) comprend en outre la mesure d'au moins un marqueur biologique choisi parmi le groupe consistant en :

- les marqueurs de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53; et
- les marqueurs de la barrière épidermique, ledit marqueur de la barrière épidermique étant de préférence choisi parmi le groupe consistant en la filaggrine, la kératine 1, la scielline, la lorcrine, la claudine 1, barx2; et
- les marqueurs de l'adhésion et de la réparation cutanée, ledit marqueur de l'adhésion et de la réparation cutanée étant de préférence choisi parmi la molécule d'adhésion cellulaire 1, la molécule CD36, la fibronectine 1, et
- les marqueurs préférentiellement exprimés dans les cellules souches, ledit marqueur préférentiellement exprimé

dans les cellules souches étant de préférence choisi parmi la protéine suppresseur de tumeur p63, la survivine.

Il sera par ailleurs évident à l'homme du métier que la méthode permettra une évaluation de l'efficacité de la formulation ou de l'actif d'autant plus complète qu'un grand nombre de marqueurs de types différents seront utilisés.

En particulier, le procédé est caractérisé en ce que l'étape c) comprend la mesure du niveau d'expression d'une combinaison de marqueurs biologiques comprenant ou consistant en :

- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53 ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2 ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur préférentiellement exprimé dans les cellules souches, ledit marqueur préférentiellement exprimé dans les cellules souches étant de préférence choisi parmi la protéine suppresseur de tumeur p63 et la survivine ; ou

En particulier, le procédé de est caractérisé en ce que l'étape c) comprend la mesure du niveau d'expression d'une combinaison de marqueurs biologiques comprenant ou consistant en

- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53, et au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1 ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53, et au moins un marqueur préférentiellement exprimé dans les cellules souches, ledit marqueur préférentiellement exprimé dans les cellules souches étant de préférence choisi parmi la protéine suppresseur de tumeur p63 et la survivine ; ou
- au moins un marqueur de l'inflammation cutanée, où le marqueur de l'inflammation cutanée est de préférence choisi

parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1 ; ou

- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2, et au moins un marqueur préférentiellement exprimé dans les cellules souches, ledit marqueur préférentiellement exprimé dans les cellules souches étant de préférence choisi parmi la protéine suppresseur de tumeur p63 et la survivine ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1, et au moins un marqueur préférentiellement exprimé dans les cellules souches, ledit marqueur préférentiellement exprimé dans les cellules souches étant de préférence choisi parmi la protéine suppresseur de tumeur p63 et la survivine.

En particulier, le procédé de est caractérisé en ce que l'étape c) comprend la mesure du niveau d'expression d'une combinaison de marqueurs biologiques comprenant ou consistant en

- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53, et au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1 ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1, et au moins un marqueur préférentiellement exprimé dans les cellules souches, ledit marqueur préférentiellement exprimé dans les cellules souches étant de préférence choisi parmi la protéine suppresseur de tumeur p63 et la survivine ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine β4, la protéine suppresseur de tumeur p53, et au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2, et au moins un marqueur préférentiellement exprimé dans les cellules souches, ledit marqueur préférentiellement exprimé dans les cellules souches étant de préférence choisi parmi la protéine suppresseur de tumeur p63 et la survivine ; ou
- au moins un marqueur de l'inflammation cutanée, où le marqueur de l'inflammation cutanée est de préférence choisi parmi le groupe consistant en PTGS2, les interleukines, de préférence IL1α et IL8, le récepteur à l'IL1 : IL1-R, l'antagoniste au récepteur à l'IL1 : IL1-RN, les métalloprotéases matricielles, de préférence MMP1 a et MMP3, et

au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2, et au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1, et au moins un marqueur préférentiellement exprimé dans les cellules souches, ledit marqueur préférentiellement exprimé dans les cellules souches étant de préférence choisi parmi la protéine suppresseur de tumeur p63 et la survivine.

L'utilisation de combinaisons de marqueurs comprenant au moins un marqueur de chacun des différents types indiqués plus haut est particulièrement avantageuse.

Ainsi, de préférence, le procédé de est caractérisé en ce que l'étape c) comprend la mesure du niveau d'expression d'une combinaison de marqueurs biologiques comprenant ou consistant en :

- au moins un marqueur de l'inflammation cutanée, où le marqueur de l'inflammation cutanée est de préférence choisi parmi le groupe consistant en PTGS2, les interleukines, de préférence IL1$\alpha$ et IL8, le récepteur à l'IL1 : IL1-R, l'antagoniste au récepteur à l'IL1 : IL1-RN, les métalloprotéases matricielles, de préférence MMP1 a et MMP3 ; et
- au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine $\beta$4, la protéine suppresseur de tumeur p53; et

- au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2; et
- au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1 ; et
- au moins un marqueur préférentiellement exprimé dans les cellules souches, ledit marqueur préférentiellement exprimé dans les cellules souches étant de préférence choisi parmi la protéine suppresseur de tumeur p63 et la survivine.

Les inventeurs ont en particulier montré que l'utilisation de l'ensemble des marqueurs du procédé permet d'évaluer de manière très précise l'efficacité d'une formulation ou d'un principe actif dans la prévention des effets délétères des UV sur la peau d'enfant âgé de trois ans ou moins.

Ainsi, de préférence, le procédé est caractérisé en ce que l'étape c) comprend la mesure du niveau d'expression d'une combinaison de marqueurs biologiques comprenant ou consistant en PTGS2, IL1$\alpha$, IL8, IL1-R, IL1-RN, MMP1, MMP3, la catalase, la superoxide dismutase 1, la défensine $\beta$4, la protéine suppresseur de tumeur p53, la filaggrine, la kératine 1, la scielline, la lorcrine, la claudine 1, barx2, la molécule d'adhésion cellulaire 1, la molécule CD36, la fibronectine 1, la protéine suppresseur de tumeur p63 et la survivine.

Pour chacun de ces marqueurs, les termes « niveau d'expression » se réfèrent préférentiellement au niveau de synthèse d'au moins un des produits du gène dudit marqueur.

Plus précisément, le niveau d'expression dudit marqueur biologique correspond à la quantité ou à la concentration cellulaire du transcrit dudit gène ou de la protéine issue dudit transcrit. Par exemple, le niveau d'expression dudit marqueur biologique correspond à la quantité ou à la concentration cellulaire du transcrit dudit gène. Par exemple, le niveau d'expression dudit marqueur biologique correspond à la quantité ou à la concentration cellulaire de la protéine issue dudit transcrit.

Par la « mesure du niveau d'expression d'une combinaison de marqueurs biologiques », on entend ici la mesure du niveau d'expression de chacun des marqueurs de la combinaison. L'expression d'un gène peut être mesurée par exemple au niveau nucléotidique, en mesurant la quantité de transcrits dudit gène, et peut aussi être mesurée par exemple au niveau peptidique, en mesurant par exemple la quantité des protéines issus desdits transcrits. Ainsi, par « mesure du niveau d'expression dudit gène » on entend ici la mesure de la quantité de produit du gène sous sa forme peptidique ou sous sa forme nucléotidique.

Ainsi, le procédé peut comprendre une ou plusieurs étapes préalable(s) à la mesure de l'expression du marqueur biologique, lesdites étapes correspondant à l'extraction à partir du modèle de peau de mamelon de l'étape a), d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc) ou de protéines à partir d'un tissu tel qu'un modèle de peau ou de cellules ne sont que des procédures de routine bien connues de l'homme du métier.

Pour chacun des types de marqueurs biologiques du procédé, de nombreux procédés sont à la disposition de l'homme du métier pour mesurer le niveau d'expression dudit marqueur biologique.

Quand le niveau d'expression du marqueur est mesuré au niveau nucléotidique, c'est-à-dire en mesurant la

quantité de produit du gène sous sa forme nucléotidique, n'importe quelle technologie habituellement utilisée par l'homme du métier pour mesurer des quantités de nucléotides peut être mise en oeuvre. Les procédés d'analyse du niveau d'expression des gènes au niveau nucléotidique, comme par exemple l'analyse du transcriptome, incluent des procédés bien connues telles que la RT-PCR ou la RT-PCR quantitative ou encore les puces d'acides nucléiques. Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose. Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides. Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Préférentiellement, le procédé est mis en oeuvre en utilisant toute méthode actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676- 684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand le niveau d'expression du marqueur est mesuré au niveau peptidique, c'est-à-dire en mesurant la quantité de produit du gène sous sa forme peptidique, toute méthode pour déterminer le niveau d'expression d'un polypeptide connue de l'homme du métier peut être utilisée. Les méthodes pour déterminer le niveau d'expression d'un polypeptide incluent par exemple la spectrométrie de masse, les tests biochimiques, y compris les tests immunologiques tels que par exemple les tests immunologiques de détection classiques (les tests ELISA et les tests ELISPOTS), ou encore comme par exemple des tests immunologiques employant des techniques de transfert des protéines sur support, tels que le slot blot (aussi appelé dot blot) ou le western blot. Il est par exemple possible d'employer les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Par «un niveau d'expression de référence d'un marqueur biologique », on entend ici tout niveau d'expression dudit marqueur utilisé à titre de référence. Par exemple, un niveau d'expression de référence peut être obtenu en mesurant le niveau d'expression du marqueur d'intérêt dans un modèle de peau comprenant des kératinocytes obtenus à partir de sujets âgés de trois ans ou moins, dans des conditions particulières. L'homme du métier saura choisir ces conditions particulières en fonction de son objectif lors de la mise en oeuvre du procédé.

Par exemple, le niveau d'expression de référence d'un marqueur biologique est le niveau d'expression dudit marqueur obtenu dans un modèle de peau comprenant des kératinocytes obtenus à partir de sujets âgés de trois ans ou moins, non traité avec la formulation ou l'actif d'intérêt, et exposé aux UV.

Par exemple, le niveau d'expression de référence d'un marqueur biologique est le niveau d'expression dudit

marqueur obtenu dans un modèle de peau comprenant des kératinocytes obtenus à partir de sujets âgés de trois ans ou moins, mis en contact avec une formulation ou l'actif de référence, et exposé aux UV.

Lorsque le niveau d'expression de référence est un niveau d'expression obtenu dans un modèle de peau exposé aux rayonnements aux UV, l'homme du métier comprendra aisément que les conditions d'expositions aux UV du modèle de peau utilisé dans le procédé et du modèle utilisé pour obtenir un niveau d'expression de référence sont préférentiellement identiques. Ainsi, préférentiellement, la longueur d'onde des rayonnements UV utilisés, ainsi que la durée de l'exposition utilisés dans le procédé et du modèle utilisé pour obtenir un niveau d'expression de référence sont préférentiellement identiques.

Par exemple, l'homme du métier pourra utiliser comme formulation de référence toute formulation connue de l'art antérieur pour son effet dans la prévention des effets délétères des UV sur la peau.

Préférentiellement, la formulation de référence est choisie parmi un spray haute protection, un lait protection solaire et une crème protection solaire. Encore plus préférentiellement, le spray haute protection correspond à la formulation du Tableau 1, le lait protection solaire à celle du Tableau 2 et la crème protection solaire à celle du Tableau 3.

**Tableau 1** : Spray haute protection

| **Matière première / Nom commercial** | % |
|---|---|
| MIGLYOLGELB | 1 à 10 % |
| CAPRYLATE/CAPRATE DE COPRAH | 5 à 20% |
| DICAPRYLYLCARBONATE | 1 à 10 % |
| DIBUTYL ADIPATE | 1 à 10 % |
| CAPRYLOCAPRATE DE GLYCEROL | 1 à 10 % |
| ETHYLHEXYL TRIAZONE | 1 à 10 % |
| DIETHYLAMINO HYDROXYBENZOYL HEXYL BENZOATE | 1 à 10 % |
| BIS- ETHYLH EXYLOXYPH ENOL METHOXYPH ENYL TRIAZINE | 1 à 10 % |
| HUILE D'AVOCAT | 1 à 5% |
| ALPHA-TOCOPHEROL | 0 à 1% |
| EAU PURIFIEE | QSP 100 |
| GLYCEROL | 1 à 5% |
| GOMME XANTHANE | 0 à 1% |
| POTASSIUMCETYLPHOSPHATE | 0 à 2% |
| ARGININE | 0 à 2% |
| PHENYLBENZIMIDAZOLE SULFONIC ACID | 1 à 5% |
| CONSERVATEURS | 0 à 2% |
| PERSEOSE AVOCAT | 0 à 1% |
| LAURYLGLUCOSE-GLYSTEARATE | 1 à 10 % |

**Tableau 2** : Lait protection solaire

| **Matière première / Nom commercial** | % |
|---|---|
| MIGLYOLGELB | 1 à 10 % |
| CAPRYLATE/CAPRATE DE COPRAH | 5 à 20% |
| DICAPRYLYLCARBONATE | 1 à 10 % |
| DIBUTYL ADIPATE | 1 à 10 % |
| CAPRYLOCAPRATE DE GLYCEROL | 1 à 10 % |
| ETHYLHEXYL TRIAZONE | 1 à 10 % |

(suite)

| Matière première / Nom commercial | % |
|---|---|
| DIETHYLAMINO HYDROXYBENZOYL HEXYL BENZOATE | 1 à 10 % |
| BIS-ETHYLH EXYLOXYPH ENOL METHOXYPHENYL TRIAZINE | 1 à 10 % |
| TITANIUM DIOXIDE | 1 à 10 % |
| HUILE D'AVOCAT | 1 à 5% |
| ALPHA-TOCOPHEROL | 0 à 1% |
| EAU PURIFIEE | QSP 100 |
| GLYCEROL | 1 à 5% |
| GOMMEXANTHANE | 0 à 1% |
| POTASSIUMCETYLPHOSPHATE | 0 à 2% |
| ARGININE | 0 à 2% |
| PHENYLBENZIMIDAZOLE SULFONIC ACID | 1 à 5% |
| CONSERVATEURS | 0 à 2% |
| PERSEOSE AVOCAT | 0 à 1% |
| LAURYLGLUCOSE-GLYSTEARATE | 1 à 10 % |

**Tableau 3** : Crème protection solaire

| Matière première / Nom commercial | % |
|---|---|
| EAU PURIFIEE | QSP 100 |
| TITANIUM DIOXIDE | 1 à 25% |
| CAPRYLATE/CAPRATE DE COPRAH | 1 à 10% |
| DICAPRYLYL CARBONATE | 1 à 10% |
| MIGLYOL GEL | 1 à 10% |
| CAPRYLOCAPRATE DE GLYCEROL | 1 à 10% |
| POLYGLY2DIPOLYHYDRSTEARATE | 1 à 10% |
| POLYGLYCERYL3DIISOSTEARATE | 1 à 10% |
| HUILE D'AVOCAT | 1 à 5% |
| GLYCEROL | 1 à 5% |
| CONSERVATEURS | 1 à 2% |
| SULFATE DE MAGNESIUM | 1 à 2% |
| ALPHA-TOCOPHEROL | 0 à 1% |
| GOMMEXANTHANE | 0 à 1% |
| ARGININE | 0 à 1% |
| PERSEOSE AVOCAT | 0 à 1% |

De même, l'homme du métier pourra utiliser comme actif de référence tout actif connu de l'art antérieur pour son effet dans la prévention des effets délétères des UV sur la peau.

En particulier, l'homme du métier pourra utiliser le perséose d'avocat, dont il a été montré précédemment qu'il permet de prévenir les effets délétères des rayons UV (voir la demande FR 1351136).

Par perséose d'avocat, on se réfère ici à un sucre en C7 ou dérivé, ou à un mélange de sucres en C7 de

formule (I) suivante :

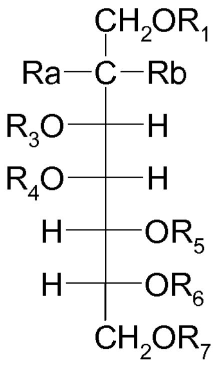

(I)

dans laquelle

Ra représente un atome d'hydrogène et Rb représente un $-OR_2$ ou CRaRb représente le radical CO ;

$R_1$, $R_2$, $R_3$, $R_4$, $R_5$, $R_6$ et $R_7$ représentent, indépendamment l'un de l'autre

- un atome d'hydrogène ou
- un radical -(CO)-R dans lequel R représente une chaine hydrocarbonée saturée ou insaturée contenant de 11 à 24 atomes de carbone, éventuellement substituée par un ou plusieurs substituants choisis dans le groupe constitué par les radicaux hydroxy (-OH), les radicaux éthoxy ($-OC_2H_5$) et groupement $-SO_3M$ avec M représentant un atome d'hydrogène, un ion ammonium $NH_4^+$ ou un ion métallique ; ou
- un radical -(CO)-R' dans lequel R' représente une chaine hydrocarbonée saturée ou insaturée contenant de 2 à 10 atomes de carbone, éventuellement substituée par un ou plusieurs substituants choisis dans le groupe constitué par les radicaux hydroxy (-OH), les radicaux éthoxy ($-OC_2H_5$) et groupement - $SO_3M$ avec M représentant un atome d'hydrogène, un ion ammonium $NH_4^+$ ou un ion métallique.

L'actif de référence peut être également choisi parmi des actifs classiquement utilisés en dermatologie ou cosmétique tels que les émollients, les actifs hydratants, les agents restructurant de la barrière cutanée, les agonistes PPARs (ou Peroxysome Proliferator Activated Receptor), les agonistes RXR ou LXR, les agents cicatrisants, les agents anti-irritants, les agents apaisants, les agents anti-inflammatoires, les agents antioxydants, les filtres et écrans solaires minéraux ou organiques, les composés antifongiques, les agents antibactériens, les conservateurs.

Plus particulièrement, les agents cicatrisants et/ou restructurants de la barrière cutanée pouvant être utilisés sont avantageusement le panthénol (vitamine B5), l'arabinogalactane, l'oxyde de zinc, les céramides, le cholestérol, le squalane et les phospholipides.

L'agent anti-inflammatoire et/ou anti-irritant et/ou apaisant peut être l'arabinogalactane ou l'oléodistillat de tournesol.

Les actifs protecteurs solaires pouvant être utilisés sont avantageusement des filtres et écrans solaires UVB et/ou UVA, tels les écrans ou filtres minéraux et/ou organiques connus de l'homme du métier qui adaptera leur choix et leurs concentrations en fonction du degré de protection recherché.

Les conservateurs pouvant être utilisés sont par exemple ceux généralement utilisés en cosmétique, les molécules à activité antibactérienne (pseudo-conservateurs) tels que les dérivés capryliques comme par exemple le capryloyl glycine et le glycéryl caprylate ; l'hexanediol, le sodium levulinate, et les dérivés de zinc et de cuivre (gluconate et PCA).

L'actif de référence peut être en particulier choisi parmi les extraits végétaux, en particulier :

- les huiles végétales telles que l'huile de soja et/ou l'huile de colza, l'huile d'avocat (WO2004/012496, WO2004/012752, WO2004/016106, WO2007/057439), l'huile de lupin et avantageusement l'huile de lupin blanc doux (WO98/47479), ou un mélange de ces huiles ;

- l'oléodistillat ou les concentrats d'huile végétale ou animale, notamment de tournesol, plus avantageusement des concentrats de tournesol linoléiques, tels que l'huile de tournesol concentrée en insaponifiables (Soline® - WO2001/21150), commercialisée par les Laboratoires Expanscience, les huiles concentrées en insaponifiables du

type huile d'avocat, de colza, de maïs utiles notamment pour leur activité hydratante et/ou émolliente, cicatrisante et/ou restructurante de la barrière cutanée, anti-inflammatoire et/ou anti-irritante et/ou apaisante ;

- Les insaponifiables de végétaux ou d'huile végétale, avantageusement des furanes d'avocat (Avocadofurane®), pouvant être obtenus par le procédé décrit dans la demande internationale WO 01/21605 , les insaponifiables d'Avocat et/ou de Soja, plus particulièrement un mélange d'insaponifiables d'Avocat furaniques et d'insaponifiables de soja, avantageusement dans un rapport respectif d'environ 1/3 - 2/3 (tel que Piasclédine®), les insaponifiables de soja (tels qu'obtenus selon le procédé décrit dans le demande internationale WO 01/51596), les insaponifiables stéroliques (typiquement des insaponifiables dont la teneur en stérols, en méthylstérols et en alcool triterpéniques est comprise entre 20 et 95% en poids, de préférence 45-65% en poids, par rapport au poids total de l'insaponifiable), les phytostérols, les esters de stérols et les dérivés vitaminiques, utiles notamment pour leur activité cicatrisante et/ou restructurante de la barrière cutanée et/ou anti-inflammatoire ;

- Les peptides ou complexes d'acides aminés végétaux, en particulier les d'avocat (tel que ceux décrits dans la demande internationale WO2005/105123), les peptides de lupin (tel que ceux décrits dans la demande internationale WO 00/62789), l'extrait total de lupin (tel que ceux décrits dans la demande internationale WO2005/102259), les peptides de quinoa (tel que ceux décrits dans la demande internationale WO2008/080974), les peptides de maca (tel que ceux décrits dans la demande internationale WO2004/112742), les peptides de soja fermenté ou non, les peptides de riz (tel que ceux décrits dans la demande internationale WO2008/009709), utiles notamment pour leur activité hydratante et/ou émolliente (avocat), kératorégulatrice (lupin, quinoa), cicatrisante et/ou restructurante de la barrière (maca, quinoa, soja), anti-inflammatoire et/ou anti-irritante et/ou apaisante (lupin, quinoa), antioxydante (avocat), les peptides de schizandra (tel que ceux décrits dans la demande de brevet FR 0955344), l'extrait de graines d'*Acacia macrostachya* (tels que celui décrit dans la demande de brevet FR 0958525), l'extrait de graines de *Vigna unguiculata* (tels que celui décrit dans la demande de brevet FR 0958529) ; extrait de graines de passiflore (tels que celui décrit dans la demande de brevet FR 1262234) ;

- Les extraits riches en polyphénols, et plus particulièrement les extraits de fruits d'avocat (tels que ceux décrits dans la demande FR 1 061 055), les extraits de feuilles de maca (tels que ceux décrits dans la demande FR 1 061 047), et les extraits de parties aériennes de *Gynandropsis gynandra* (tels que ceux décrits dans la demande FR 1 061 051),

- Le lupéol (FR 2 8 22 821, FR 2 857 596) utile notamment pour favoriser la cicatrisation ;

- Un beurre de Cupuaçu, particulièrement apprécié pour ses propriétés hydratantes.

L'actif de référence peut aussi être plus particulièrement choisi parmi les oxazolines, en particulier celles choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline (de préférence la 2-undécyl-4,4-diméthyl-1,3-oxazoline, appelée OX-100 ou Cyclocéramide® ; WO 2004/050052, WO 2004/050079, et WO 2004/112741). Elles sont particulièrement utiles pour leur activité anti-inflammatoire et/ou anti-irritante et/ou apaisante, antioxydante.

Les composés protecteurs ou activateurs des cellules souches comme Stemoxydine® (diethyl pyridine-2,4-dicarboxylate), Survicode™ (sodium cocoyl alaninate), Survixyl IS™ (pentapeptide-31),Defensil® (ctyldodecanol, Echium Planta-gineum Seed Oil, Cardiospermum Halicacabum Flower/Leaf/Vine Extract, Helianthus Annuus Sun-flower Seed Oil Unsaponifiables), Celligent® (Helianthus Annuus Sunflower Seed Oil, Ethyl Ferulate, Polyglyceryl-5 Trioleate, Rosmarinus Officinalis Leaf Extract, Aqua, Disodium Uridine Phosphate),Phycosaccharide AI®(alginic acid, sodium salt, hydrolysed), Phycojuvenine® (Laminaria digitata extract), PhytoCellTec™ à base d'extrait de cellules souches végétales de rose blanche des Alpes ou de raisin Gamay teinturier Fréaux ou de pomme Uttwiler spätlauber (Malus domestica) ou d'argan, cellules souches végétales extraites de vignes Vitis vinifera, cellules souches végétales de jeunes pousses de Christe Marine peuvent aussi être des actifs de référence au sens du procédé.

Par exemple, le niveau d'expression de référence d'un marqueur biologique est le niveau d'expression dudit marqueur obtenu dans un modèle de peau comprenant des kératinocytes obtenus à partir de sujets âgés de trois ans ou moins, préférablement de deux ans ou moins, plus préférablement d'un an ou moins, encore plus préférablement de six mois ou moins, et en particulier de trois mois ou moins, non traité avec la formulation ou l'actif d'intérêt, et non exposé aux UV.

Par exemple, le niveau d'expression de référence d'un marqueur biologique est le niveau d'expression dudit marqueur obtenu dans un modèle de peau comprenant des kératinocytes obtenus à partir de sujets âgés de trois ans ou moins, préférablement de deux ans ou moins, plus préférablement d'un an ou moins, encore plus préférablement

de six mois ou moins, et en particulier de trois mois ou moins, traité avec la formulation ou l'actif d'intérêt, et non exposé aux UV.

L'homme du métier comprendra en outre aisément que la comparaison de l'étape d) est de préférence effectuée entre des mesures de niveaux d'expression obtenus pour des modèles de peau comprenant des kératinocytes obtenus à partir de sujets âgés de trois ans ou moins, préférablement de deux ans ou moins, plus préférablement d'un an ou moins, encore plus préférablement de six mois ou moins, et en particulier de trois mois ou moins, de structures histologiques similaires, voire identiques. Par « structures histologiques similaires », on entend ici que les proportions relatives des types cellulaires compris dans les modèles de peau comparés sont similaires. Ainsi, il est préférable que les proportions relatives des types cellulaires compris dans le modèle de peau de l'étape a) ne diffèrent pas de plus de 5% des proportions relatives des types cellulaires compris dans le modèle de peau utilisé pour l'obtention du niveau d'expression de référence de l'étape d). Par « proportion relative d'un type cellulaire » on entend ici le rapport du nombre de cellules correspondant à ce type cellulaire sur le nombre de cellules totales comprises dans le modèle de peau. Ainsi, il est par exemple préférable que la proportion de kératinocytes sur le nombre de cellules totales dans le modèle de peau de l'étape a) ne diffère pas de plus de 5% de la proportion de kératinocytes sur le nombre de cellules totales dans le modèle de peau utilisé pour l'obtention du niveau d'expression de référence de l'étape d). Par « structures histologiques identiques », on entend ici que les proportions relatives des types cellulaires compris dans les modèles de peau comparés sont identiques. Au sens du présent procédé, les proportions relatives des types cellulaires compris dans le modèle de peau de mamelon de l'étape a) sont identiques aux proportions relatives des types cellulaires compris dans le modèle de peau utilisé pour l'obtention du niveau d'expression de référence de l'étape d) lorsqu'elles ne diffèrent pas de plus de 0,1%. Avantageusement, la proportion de kératinocytes sur le nombre de cellules totales dans le modèle de peau de l'étape a) ne diffère pas de plus de 0,1% de la proportion de kératinocytes sur le nombre de cellules totales dans le modèle de peau utilisé pour l'obtention du niveau d'expression de référence de l'étape d).

L'homme du métier comprendra tout aussi aisément que la comparaison de l'étape d) est de préférence effectuée entre des mesures de niveaux d'expression obtenus pour des modèles de peau qui sont de taille, de volume ou de poids similaires, voire identiques. Ainsi, il est préférable que la taille, le volume, ou le poids du modèle de peau de l'étape a) ne diffère pas de plus de 5% de la taille, du volume, ou du poids du modèle de peau utilisé pour l'obtention du niveau d'expression de référence de l'étape d). Plus préférentiellement, la taille, et le volume et le poids du modèle de peau de l'étape a) ne diffèrent pas de plus de 5% de la taille, du volume et du poids du modèle de peau utilisé pour l'obtention du niveau d'expression de référence de l'étape c). Encore plus préférentiellement, la taille, et le volume et le poids du modèle de peau de l'étape a) ne diffèrent pas de plus de 0,1% de la taille, du volume et du poids du modèle de peau utilisé pour l'obtention du niveau d'expression de référence de l'étape d).

Alternativement, si les modèles de peau diffèrent de plus de 5 % de par la taille, le volume et le poids, l'homme du métier pourra normaliser le niveau obtenu à l'étape c) et le niveau de référence de l'étape d) à l'aide d'un facteur de normalisation.

Ce facteur de normalisation pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau d'expression d'un gène qui est exprimé au même niveau dans toutes les cellules, ou presque, de l'organisme. En d'autres termes, en particulier, on utilise comme facteur de normalisation le niveau d'expression d'un gène de ménage. Par exemple, le niveau obtenu à l'étape c) et le niveau de référence de l'étape d) sont normalisés en utilisant le niveau d'expression, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genetics 19: 362-365, 2003). Les gènes de ménage selon le procédé incluent par exemple les gènes B2M, TFRC, YWHAZ, RPLO, 18S, GUSB, UBC, TBP, GAPDH, PPIA, POLR2A, ACTB, PGK1, HPRT1, IPO8 et HMBS.

L'homme du métier pourra ainsi aisément évaluer l'efficacité de la formulation d'intérêt en fonction de la comparaison de l'étape d).

Par exemple, lorsque le niveau d'expression de PTGS2, d'IL1, d'IL8, d'IL1-R, d'IL1-RN, de MMP1a ou de MMP3 mesuré à l'étape c), est inférieur ou égal au niveau d'expression de ces marqueurs obtenu dans un modèle de peau non traité avec la formulation d'intérêt, alors la formulation, ou l'actif le cas échéant, est efficace pour prévenir des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins.

Par exemple, lorsque le niveau d'expression de PTGS2, d'IL1, d'IL8, d'IL1-R, d'IL1-RN, de MMP1a ou de MMP3 mesuré à l'étape c), est égal ou n'est pas supérieur de plus de 10% au niveau d'expression de ces marqueurs obtenu dans un modèle de peau non exposé aux UV, alors la formulation est efficace pour prévenir des effets délétères des UV sur la peau d'enfant âgé de trois ans ou moins, préférablement de deux ans ou moins, plus préférablement d'un

an ou moins, encore plus préférablement de six mois ou moins, et en particulier de trois mois ou moins.

Il est ici décrit un kit pour la mise en oeuvre du procédé, comprenant les moyens nécessaires à la mesure du niveau d'expression d'au moins un marqueur de l'inflammation cutanée, où le marqueur de l'inflammation cutanée est de préférence choisi parmi le groupe consistant en PTGS2, les interleukines, de préférence IL1$\alpha$ et IL8, le récepteur à l'IL1 : IL1-R, l'antagoniste au récepteur à l'IL1 : IL1-RN, les métalloprotéases matricielles, de préférence MMP1 et MMP3.

En particulier, le kit comprend en outre les moyens nécessaires à la mesure du niveau d'expression d'au moins un marqueur biologique choisi parmi le groupe consistant en :

- les marqueurs de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine $\beta$4, la protéine suppresseur de tumeur p53; et
- les marqueurs de la barrière épidermique, ledit marqueur de la barrière épidermique étant de préférence choisi parmi le groupe consistant en la filaggrine, la kératine 1, la scielline, la lorcrine, la claudine 1, barx2; et
- les marqueurs de l'adhésion et de la réparation cutanée, ledit marqueur de l'adhésion et de la réparation cutanée étant de préférence choisi parmi la molécule d'adhésion cellulaire 1, la molécule CD36, la fibronectine 1, et
- les marqueurs préférentiellement exprimés dans les cellules souches, ledit marqueur préférentiellement exprimé dans les cellules souches étant de préférence choisi parmi la protéine suppresseur de tumeur p63, la survivine.

Préférentiellement, le kit comprend les moyens nécessaires à la mesure du niveau d'expression d'une combinaison de marqueurs biologiques comprenant ou consistant en :

- au moins un marqueur de l'inflammation cutanée, où le marqueur de l'inflammation cutanée est de préférence choisi parmi le groupe consistant en PTGS2, les interleukines, de préférence IL1$\alpha$ et IL8, le récepteur à l'IL1 : IL1-R, l'antagoniste au récepteur à l'IL1 : IL1-RN, les métalloprotéases matricielles, de préférence MMP1 a et MMP3, et
- au moins un marqueur de la défense antioxydante antimicrobienne ou de l'ADN, ledit marqueur de la défense antioxydante ou antimicrobienne étant de préférence choisi parmi le groupe consistant en la catalase, la superoxide dismutase 1, les défensines, en particulier la défensine $\beta$4, la protéine suppresseur de tumeur p53; et
- au moins un marqueur de la barrière épidermique, ledit marqueur de la barrière épidermique étant de préférence choisi parmi le groupe consistant en la filaggrine, la kératine 1, la scielline, la lorcrine, la claudine 1, barx2; et
- au moins un marqueur de l'adhésion et de la réparation cutanée, ledit marqueur de l'adhésion et de la réparation cutanée étant de préférence choisi parmi la molécule d'adhésion cellulaire 1, la molécule CD36, la fibronectine 1, et
- au moins un marqueur préférentiellement exprimé dans les cellules souches, ledit marqueur préférentiellement exprimé dans les cellules souches étant de préférence choisi parmi la protéine suppresseur de tumeur p63, la survivine.

Avantageusement, le kit comprend les moyens nécessaires à la mesure du niveau d'expression d'une combinaison de marqueurs biologiques comprenant ou consistant en PTGS2, IL1$\alpha$, IL8, IL1-R, 1L1-RN, MMP1, MMP3, la catalase, la superoxide dismutase 1, la défensine $\beta$4, la protéine suppresseur de tumeur p53, la filaggrine, la kératine 1, la scielline, la lorcrine, la claudine 1, barx2, la molécule d'adhésion cellulaire 1, la molécule CD36, la fibronectine 1, la protéine suppresseur de tumeur p63 et la survivine.

Préférentiellement, les moyens nécessaires à la mesure du niveau d'expression des marqueurs PTGS2, IL1$\alpha$, IL8, IL1-R, IL1-RN, MMP1, MMP3, la catalase, la superoxide dismutase 1, la défensine $\beta$4, la protéine suppresseur de tumeur p53, la filaggrine, la kératine 1, la scielline, la lorcrine, la claudine 1, barx2, la molécule d'adhésion cellulaire 1, la molécule CD36, la fibronectine 1, la protéine suppresseur de tumeur p63 et la survivine comprennent des sondes nucléiques et/ou des amorces d'amplification capables de se lier à au moins l'un de ces marqueurs biologiques sous sa forme nucléotidique.

Les exemples suivants sont fournis ici à titre d'illustration et ne sont pas, sauf indication contraire, destinés à être limitatifs.

## Exemples

### 1. Effet des UV sur des épidermes de nourrisson et d'enfant

#### 1.1. Introduction

Dans cette étude, des épidermes reconstruits ont été réalisés avec des kératinocytes issus d'un nourrisson de 3 mois et issus d'un enfant de 11 ans

Sur ces deux modèles, l'effet d'une irradiation UV a été évalué par :

- analyse par RT-qPCR de l'expression d'une sélection de marqueurs ;
- analyse histologique des tissus et analyse de la formation des sunburn cells ;
- analyse par immunomarquage de l'expression de l'intégrine alpha 6

**1.2. Matériel et Méthodes**

Les épidermes ont été réalisés avec les kératinocytes des différents donneurs selon le modèle dérivé de la méthode de Poumay et al (Arch Dermatol Res 2004 ; 296: 203-11). Après 2 jours de culture en immersion, les épidermes reconstruits ont été cultivés à l'interface air/liquide pendant 10 jours. Toutes les conditions expérimentales ont été réalisées en duplicate, n=2.

Pour chaque lot d'épiderme, à J10, les épidermes ont été incubés pendant 24h.

Après incubation, les épidermes ont été irradiés avec une dose de 500 mJ/cm$^2$ d'UVB + 7255 mJ/cm$^2$ d'UVA. Après irradiation, les épidermes ont été incubés pendant 30 min (marquage intégrine alpha 6) ou 24 heures (analyse expression des gènes et histologie). Pour chaque âge, il y a eu également un lot non traité et non irradié et un lot non traité et irradié.

**1.2.1.** Histologie

A la fin de l'incubation, les épidermes ont été rincés puis soit congelés en isopentane azote liquide et conservés à -80°C soit fixés dans du formaldéhyde. Les tissus fixés ont ensuite été déshydratés dans des bains d'éthanol puis inclus en paraffine. Des coupes transversales ont été réalisées au microtome (5μm épaisseur, plusieurs coupes par lame, 1 lame par épiderme) puis maintenus à température ambiante jusqu'à réalisation des colorations.

Les coupes ont été déparaffinées puis colorées selon le protocole classique de coloration hématoxyline/éosine. Après rinçage, les lames ont été montées entre lames et lamelles.

Les coupes ont été observées à l'aide d'un microscope NIKON E400. Les images numériques ont été enregistrées avec une caméra NIKON DS-Ri1 et le logiciel NIS-Elements 3.10.

Les Sunburn cells ont été comptées et rapportées à la surface totale des épidermes reconstruits.

**1.2.2.** Immunomarquage in situ

A partir des épidermes congelés, des coupes transversales ont été réalisées au cryostat (5 μm épaisseur, plusieurs coupes par lame, 1 lame par épiderme). Les cryocoupes ont été fixées par un mélange acétone/méthanol et séchées à l'air ambiant. Après saturation en PBS-T-lait 5%, les coupes ont été ensuite incubées à température ambiante pendant 1 heure avec l'anticorps primaire dirigé contre l'intégrine alpha6. Après lavage au PBS, le marquage a été révélé avec un anticorps secondaire directement couplé à un fluorochrome Alexa 488. Les noyaux des cellules ont été colorés avec une solution d'iodure de propidium. Les coupes ont été lavées et montées entre lames et lamelles en milieu aqueux.

Les coupes ont été observées à l'aide d'un microscope NIKON E400. Les images numériques ont été enregistrées avec une caméra NIKON DS-Ri1 et le logiciel NIS-Elements 3.10. Les intensités de fluorescence ont été mesurées avec le logiciel ImageJ.

**1.2.3.** Analyse de l'expression différentielle des gènes

L'expression des marqueurs a été évaluée par RT-qPCR sur les ARN messagers extraits des épidermes reconstruits de chaque traitement.

L'analyse de l'expression des gènes a été réalisée en n=2 à l'aide d'un PCR array (mQPA-STRESS-UV-64) contenant 62 gènes d'intérêt et 2 gènes de référence (housekeeping genes).

Les ARN totaux de chaque échantillon ont été extraits à l'aide de TriPure Isolation Reagent® selon le protocole préconisé par le fournisseur. La quantité et la qualité des ARN ont été évaluées par électrophorèse capillaire (Ambion). Les ADN complémentaires (ADNc) ont été synthétisés par transcription inverse des ARN en présence d'oligo(dT) et de l'enzyme Superscript II. L'ADNc obtenu a été quantifié par spectrophotométrie, puis les ADNc ont été ajustés à 10 ng/μl.

Les réactions de PCR ont été réalisées par PCR quantitative avec le système « light cycler » (Roche Molecular System Inc) et selon la procédure recommandée par le fournisseur.

Le mélange réactionnel pour chaque échantillon a été : ADNc 10 ng/μl, amorces des différents marqueurs utilisés, mélange réactionnel contenant l'enzyme taq DNA polymérase, le marqueur SYBR Green I (agent intercalant de l'ADN) et du $MgCl_2$.

L'incorporation de fluorescence dans l'ADN amplifié est mesurée en continu au cours de cycles de PCR. Ces mesures permettent d'obtenir des courbes d'intensité de la fluorescence en fonction des cycles de PCR et d'évaluer

ainsi une valeur d'expression relative pour chaque marqueur. Le nombre de cycles est déterminé à partir des points de « sortie » des courbes de fluorescence. Pour un même marqueur analysé, plus un échantillon « sort » tard (nombre de cycle élevé), plus le nombre initial de copies de l'ARN est faible.

La valeur d'expression relative est exprimée en unité arbitraire (UA) selon la formule suivante :

$$(1/2^{\text{nombre de cycles}}) X 10^6).$$

L'expression des gènes d'intérêt est normalisée par les expressions des 2 gènes de référence

Puis les pourcentages d'expression des gènes dans les épidermes irradiés sont calculés par rapport à leur expression dans les épidermes non irradiés (témoin non irradié) fixée à 100% (Tableau 5).

**Tableau 4** : Classification des gènes :

| Nom du cluster | Abréviation | Nom du gène |
|---|---|---|
| Housekeeping | RPS28 | Ribosomal protein 28S |
| | GADPH | Glyceraldehyde-3-phosphate dehydrogenase |
| Inflammation | IL1A | Interleukin 1, alpha |
| | IL1RN | Interleukin 1 receptor antagonist |
| | IL8 | Interleukin 8 |
| | PTGS2 | Prostaglandin-endoperoxidase synthase 2 (prostaglandin G/H synthase and cyclooxygenase) |
| | MMP1 | Matrix metallopeptidase 1 |
| | MMP3 | Matrix metallopeptidase 1 |
| Défense antioxydante, antimicrobienne, protection ADN | CAT | Catalase |
| | SOD1 | Superoxide dismutase 1, soluble |
| | DEFB4 | Defensin, beta 4 |
| | TP53 | Tumor protein p53 |
| Différenciation épiderme, Fonction barrière | FLG | Filaggrin |
| | KRT1 | Keratin 1 |
| | SCEL | Sciellin |
| | LOR | Loricrin |
| | CLDN1 | Claudin 1 |
| | BARX2 | BARX homeobox 2 |
| Interaction cellule-cellule, adhésion matrice et réparation épiderme | CADM1 | Cell adhesion molecula 1 |
| | CD36 | CD36 molecule (thrombospondin receptor) |
| | FN1 | Fibronectin 1 |
| Cellules souches | TP63 | Tumor protein p63 |
| | BIRC5 | Baculoviral IAP repeat-containing 5 or survivin |

### 1.3. Résultats

#### 1.3.1. Histologie

Les épidermes reconstruits avaient une morphologie attendue à J12. En effet les différentes assises cellulaires étaient présentes :

- la couche basale

- la couche épineuse
- la couche granuleuse
- la couche cornée anucléée.

Comme le montre la Figure 1, il n'y a pas de différence observée au niveau histologique entre les épidermes de 3 mois et les épidermes de 11 ans. Cependant l'irradiation UV a causé une altération nette de la couche cornée de l'épiderme de 3 mois qui se décolle du reste de l'épiderme, alors que l'épiderme et la couche cornée ne sont quasiment pas modifiés après irradiation de l'épiderme de 11 ans. L'histologie des épidermes reconstruits de 11 ans est comparable à celle d'épidermes reconstruits adultes irradiés dans les mêmes conditions.
Les sunburn cells ont été identifiées par leur morphologie caractéristique (Figure 1) : noyau basophile sombre, pycnotique et condensé, cytoplasme éosiphile et formation d'espaces intercellulaires.
Les sunburn cells sont présentes en grand nombre dans les épidermes irradiés avec un nombre toutefois supérieur dans les épidermes de 3 mois irradié.
Les épidermes reconstruits de 3 mois sont donc plus fortement altérés par l'irradiation UV que les épidermes de 11 ans.

**1.3.2.** Profil d'expression des gènes

Comme attendu, l'irradiation des épidermes reconstruits par les UV a fortement modulé bon nombre de gènes.
Les gènes de l'inflammation ont notamment été surexprimés, tandis que les gènes de défense antioxydante, antimicrobienne et de protection de l'ADN ont été réprimés. De plus l'expression des gènes de la différenciation épidermique et de la fonction barrière, ainsi que les gènes impliqués dans les interactions cellule-cellule et dans l'adhésion à la matrice ayant un rôle dans la réparation épidermique est inhibée par les UV.
Enfin, les gènes des cellules souches ont eu une expression fortement inhibée 24h après l'irradiation.
Les épidermes reconstruits de 3 mois ont été plus sensibles à l'irradiation des UV avec un grand nombre de gènes dont la modulation (inhibition ou stimulation) a été bien plus marquée que dans les épidermes de 11 ans irradiés (Tableau 5).

**Tableau 5** : % stimulation ou répression des gènes d'intérêt dans les épidermes irradiés par rapport aux épidermes non irradiés

| Gènes | 3 mois | 11 ans |
|---|---|---|
| | % stimulation | |
| IL1A | 2897 | 1679 |
| IL1RN | 813 | 476 |
| IL8 | 1660 | 1112 |
| PTGS2 | 2361 | 307 |
| MMP1 | 2141 | 390 |
| MMP3 | 4565 | 261 |
| | % répression | |
| CAT | 66 | 52 |
| SOD1 | 65 | 52 |
| DEFB4 | 94 | 75 |
| TP53 | 73 | 42 |
| FLG | 75 | 59 |
| KRT1 | 71 | 29 |
| LOR | 67 | 21 |
| SCEL | 54 | 0 |
| CLDN1 | 35 | 17 |
| BARX2 | 79 | 15 |

(suite)

| | % répression | |
|---|---|---|
| CADM1 | 58 | 41 |
| CD36 | 59 | 50 |
| FN1 | 73 | 0 |
| TP63 | 69 | 56 |
| BIRC5/SURVIVIN | 73 | 56 |

Les gènes de l'inflammation ont une surexpression plus importante dans les épidermes irradiés de 3 mois (Figure 2), montrant une plus grande réactivité au soleil

Les gènes de défense ont une répression plus importante dans les épidermes irradiés de 3 mois (Figure 3), montrant une plus faible capacité à se défendre. Les dommages causés par les UV sont moins facilement réparables.

Les gènes de la barrière ont une répression plus importante dans les épidermes irradiés de 3 mois (Figure 4), indiquant une plus faible protection vis-à-vis de l'environnement. Les dommages causés par les UV sont moins facilement réparables.

Les gènes de la réparation épidermique ont une répression plus importante dans les épidermes irradiés de 3 mois (Figure 5), montrant une plus faible capacité à réparer, cicatriser la peau après UV.

Les gènes des cellules souches épidermiques ont une répression plus importante dans les épidermes irradiés de 3 mois (Figure 6), montrant une plus faible capacité à régénérer la peau et à maintenir un bon équilibre.

**1.4.** Immunomarquage de l'intégrine alpha 6

La figure 7 et le tableau 6 montrent que l'expression de l'intégrine alpha 6 (marquage periplasmique) est identique dans les épidermes des deux âges mais que l'irradiation UV diminue considérablement l'intégrine alpha 6 dans les épidermes de 3 mois.

**Tableau 6** : % répression de la protéine intégrine alpha 6 dans les épidermes irradiés par rapport aux épidermes non irradiés.

| | 3 mois | 11 ans |
|---|---|---|
| Intégrine alpha 6 | 48% | 25% |

L'ensemble de ces résultats montre clairement que les épidermes reconstruits de 3 mois et les épidermes de 11 ans ne réagissent pas de la même manière face à une irradiation UV. Les effets délétères des UV sont plus importants sur les épidermes de 3 mois montrant la plus grande sensibilité de la peau des nourrissons et jeunes enfants au soleil de part une plus grande réactivité et une moindre capacité à se protéger et réparer les dommages.

## 2. Evaluation du potentiel photoprotecteur d'une crème solaire contenant du perséose d'avocat

### 2.1. Matériel et méthodes

Les épidermes ont été réalisés avec les kératinocytes isolés à partir d'un donneur de 3 mois selon le protocole décrit précédemment. Pour chaque lot d'épiderme, à J10, les épidermes ont été incubés pendant 24 h. Après incubation, les épidermes ont été traités avec une crème solaire puis ont été irradiés avec une dose de 500 mJ/cm$^2$ d'UVB + 7255 mJ/cm$^2$ d'UVA. Après irradiation, les épidermes ont été incubés pendant 24 heures. Il y a donc eu un lot non traité et non irradié, un lot non traité et irradié et un lot traité avec la crème solaire et irradié. Toutes les conditions expérimentales ont été réalisées en duplicate, n = 2.

L'analyse histologique, permettant également de compter les sunburn cells et l'analyse de l'expression différentielle des gènes ont été menées comme précédemment décrit.

### 2.2. Résultats

Comme le montre la figure 8, les effets délétères observés après irradiation des épidermes de 3 mois ne le sont plus lorsque la crème solaire a été appliquée en topique sur ces épidermes de 3 mois. Cet effet protecteur de la

crème solaire sur la structure épidermique est également montré avec la disparition des sunburn cells dont la formation avait été induite par l'irradiation.

La crème solaire a protégé nettement les épidermes reconstruits de 3 mois des dommages induits par l'irradiation UV.

De même les niveaux d'expression des gènes fortement modulés par l'irradiation UV sont régulés après application de la crème solaire.

En effet, la crème solaire appliquée sur les épidermes de 3 mois irradiés a fortement diminué l'expression des gènes de l'inflammation et a augmenté le niveau d'expression des gènes de défense, des gènes de la fonction barrière et des gènes des cellules souches (Tableau 7).

Ceci confirme l'efficacité de cette crème pour protéger la peau des nourrissons et enfants de l'exposition solaire.

Tableau 7 : Niveau d'expression des gènes de l'inflammation, de defense, de la fonction barrière et des cellules souches modulé par les UV dans les épidermes de 3 mois en l'absence ou en présence d'un produit solaire

| Gènes d'intérêt | | Expression relative de l'épiderme 3 mois irradié par rapport à l'épiderme 3 mois non irradié | Expression relative de l'épiderme 3 mois irradié en présence du produit solaire par rapport à l'épiderme 3 mois irradié |
|---|---|---|---|
| Inflammation | MMP1 | 2241 | 4 |
| | PTGS2 | 2461 | 4 |
| Défense | CAT | 34 | 112 |
| | P53 | 27 | 157 |
| Fonction Barrière | LOR | 33 | 205 |
| | BARX2 | 21 | 110 |
| Cellules Souches | TP63 | 33 | 108 |

## Revendications

**1.** Procédé d'évaluation de l'efficacité *in vitro* d'une formulation ou d'un actif sur la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins, **caractérisé en ce que** ledit procédé comprend les étapes suivantes:

a. mettre en contact ladite formulation ou ledit actif avec un modèle de peau comprenant des kératinocytes obtenus à partir de sujets âgés de 3 ans ou moins, préférablement de deux ans ou moins, plus préférablement d'un an ou moins, encore plus préférablement de six mois ou moins, et en particulier de trois mois ou moins ;
b. exposer le modèle de peau de l'étape a) aux UV ;
c. mesurer le niveau d'expression d'une combinaison de marqueurs biologiques comprenant ou consistant en :

- au moins un marqueur de l'inflammation cutanée choisi parmi le groupe consistant en PTGS2, IL1$\alpha$, IL8, le récepteur à l'IL1 : IL1-R, l'antagoniste au récepteur à l'IL1 : IL1-RN, MMP1 a et MMP3, et
- au moins un marqueur de la défense antioxydante antimicrobienne ou de l'ADN choisi parmi le groupe consistant en la catalase, la superoxide dismutase 1, la défensine $\beta$4, et la protéine suppresseur de tumeur p53; et
- au moins un marqueur de la barrière épidermique choisi parmi le groupe consistant en la filaggrine, la kératine 1, la scielline, la lorcrine, la claudine 1, et barx2; et
- au moins un marqueur de l'adhésion et de la réparation cutanée choisi parmi la molécule d'adhésion cellulaire 1, la molécule CD36, et la fibronectine 1, et
- au moins un marqueur préférentiellement exprimés dans les cellules souches choisi parmi la protéine suppresseur de tumeur p63, et la survivine,

dans le modèle de peau de l'étape b),
d. comparer le niveau d'expression d'au moins un marqueur biologique obtenu à l'étape c) avec un niveau d'expression de référence;

e. déterminer que ladite formulation ou ledit actif est efficace pour prévenir les effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins, si :

(i) le niveau d'expression d'au moins un marqueur mesuré à l'étape c), est inférieur ou égal au niveau d'expression de ce marqueur mesuré à l'étape d), lorsque ledit marqueur est un marqueur de l'inflammation cutanée ; et
(ii) le niveau d'expression d'au moins un marqueur mesuré à l'étape c), est supérieur ou égal au niveau d'expression de ce marqueur mesuré à l'étape d), lorsque ledit marqueur est un marqueur de la défense antioxydante antimicrobienne ou de l'ADN, un marqueur de la barrière épidermique, un marqueur de l'adhésion et de la réparation cutanée ou un marqueur préférentiellement exprimés dans les cellules souches.

**2.** Procédé d'identification d'une formulation ou d'un actif pour la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins, **caractérisé en ce que** ledit procédé comprend les étapes suivantes:

a. mettre en contact une formulation candidate ou un actif candidat avec un modèle de peau comprenant des kératinocytes obtenus à partir de sujets âgés de 3 ans ou moins, préférablement de deux ans ou moins, plus préférablement d'un an ou moins, encore plus préférablement de six mois ou moins, et en particulier de trois mois ou moins ;
b. exposer le modèle de peau de l'étape a) aux UV ;
c. mesurer le niveau d'expression d'une combinaison de marqueurs biologiques comprenant ou consistant en :

- au moins un marqueur de l'inflammation cutanée choisi parmi le groupe consistant en PTGS2, IL1$\alpha$, IL8, le récepteur à l'IL1 : IL1-R, l'antagoniste au récepteur à l'IL1 : IL1-RN, MMP1 a et MMP3, et
- au moins un marqueur de la défense antioxydante antimicrobienne ou de l'ADN choisi parmi le groupe consistant en la catalase, la superoxide dismutase 1, la défensine $\beta$4, et la protéine suppresseur de tumeur p53; et
- au moins un marqueur de la barrière épidermique choisi parmi le groupe consistant en la filaggrine, la kératine 1, la scielline, la lorcrine, la claudine 1, et barx2; et
- au moins un marqueur de l'adhésion et de la réparation cutanée choisi parmi la molécule d'adhésion cellulaire 1, la molécule CD36, et la fibronectine 1, et
- au moins un marqueur préférentiellement exprimés dans les cellules souches choisi parmi la protéine suppresseur de tumeur p63, et la survivine.

dans le modèle de peau de l'étape b),
d. comparer le niveau d'expression d'au moins un marqueur biologique obtenu à l'étape c) avec un niveau d'expression de référence ;
e. déterminer que ladite formulation candidate ou ledit actif candidat est une formulation ou un actif pour la prévention des effets délétères des UV sur la peau d'enfant âgé de trois ans ou moins, préférablement de deux ans ou moins, plus préférablement d'un an ou moins, encore plus préférablement de six mois ou moins, et en particulier de trois mois ou moins, si :

(i) le niveau d'expression d'au moins un marqueur mesuré à l'étape c), est inférieur ou égal au niveau d'expression de ce marqueur mesuré à l'étape d), lorsque ledit marqueur est un marqueur de l'inflammation cutanée ; et
(ii) le niveau d'expression d'au moins un marqueur mesuré à l'étape c), est supérieur ou égal au niveau d'expression de ce marqueur mesuré à l'étape d), lorsque ledit marqueur est un marqueur de la défense antioxydante antimicrobienne ou de l'ADN, un marqueur de la barrière épidermique, un marqueur de l'adhésion et de la réparation cutanée ou un marqueur préférentiellement exprimés dans les cellules souches.

**3.** Procédé selon la revendication 1 ou 2, **caractérisé en ce que** l'étape c) comprend la mesure du niveau d'expression d'une combinaison de marqueurs biologiques comprenant ou consistant en PTGS2, IL1$\alpha$, IL8, IL1-R, IL1-RN, MMP1, MMP3, la catalase, la superoxide dismutase 1, la défensine $\beta$4, la protéine suppresseur de tumeur p53, la filaggrine, la kératine 1, la scielline, la lorcrine, la claudine 1, barx2, la molécule d'adhésion cellulaire 1, la molécule CD36, la fibronectine 1, la protéine suppresseur de tumeur p63 et la survivine.

**Patentansprüche**

1. In-vitro-Verfahren zur Bewertung der Wirksamkeit einer Formulierung oder eines Wirkstoffs bei der Prävention von schädlichen Wirkungen von UV-Strahlung auf die Haut von Kindern von drei Jahren oder jünger, vorzugsweise von zwei Jahren oder jünger, mehr zu bevorzugen von einem Jahr oder jünger, noch mehr zu bevorzugen von sechs Monaten oder jünger und insbesondere von drei Monaten oder jünger, **dadurch gekennzeichnet, dass** das Verfahren die folgenden Schritte umfasst:

    a. Inkontaktbringen der Formulierung oder des Wirkstoffs mit einem Hautmodell, das Keratinozyten umfasst, die von Personen von drei Jahren oder jünger, vorzugsweise von zwei Jahren oder jünger, mehr zu bevorzugen von einem Jahr oder jünger, noch mehr zu bevorzugen von sechs Monaten oder jünger und insbesondere von drei Monaten oder jünger erhalten wurden;
    b. Exponieren des Hautmodells von Schritt a) gegenüber UV-Strahlung;
    c. Messen des Expressionsniveaus einer Kombination von biologischen Markern, die Folgendes umfasst oder aus Folgendem besteht:

        - mindestens einem Marker für Hautentzündung, der aus der Gruppe ausgewählt ist, die aus PTGS2, IL1$\alpha$, IL8, dem Rezeptor für IL1: IL1-R, dem Antagonisten für den Rezeptor für IL1: IL1-RN, MMP1 a und MMP3 besteht, und
        - mindestens einem Marker für die antimikrobielle antioxidative Abwehr oder die DNA, der aus der Gruppe ausgewählt ist, die aus Katalase, Superoxid-Dismutase 1, Defensin $\beta$4 und dem Tumorsuppressorprotein p53 besteht; und
        - mindestens einem Marker für die Hautbarriere, der aus der Gruppe ausgewählt ist, die aus Filaggrin, Keratin 1, Sciellin, Loricrin, Claudin 1 und Barx2 besteht; und
        - mindestens einem Marker für die Adhäsion und die Hautreparatur, der unter dem Zelladhäsionsmolekül 1, dem Molekül CD36 und Fibronektin 1 ausgewählt ist, und
        - mindestens einen vorzugsweise in den Stammzellen exprimierten Marker, der unter dem Tumorsuppressorprotein p63 und Survivin ausgewählt ist,

    in dem Hautmodell von Schritt b)
    d. Vergleichen des Expressionsniveaus von mindestens einem im Schritt c) erhaltenen biologischen Marker mit einem Bezugsexpressionsniveau;
    e. Bestimmen, dass die Formulierung oder der Wirkstoff für die Prävention von schädlichen Wirkungen von UV-Strahlung auf die Haut von Kindern von drei Jahren oder jünger, vorzugsweise von zwei Jahren oder jünger, mehr zu bevorzugen von einem Jahr oder jünger, noch mehr zu bevorzugen von sechs Monaten oder jünger und insbesondere von drei Monaten oder jünger wirksam ist, wenn:

        (i) das Expressionsniveau von mindestens einem im Schritt c) gemessenen Marker niedriger oder gleich dem Expressionsniveau dieses im Schritt d) gemessenen Markers ist, wenn der Marker ein Marker für Hautentzündung ist; und
        (ii) das Expressionsniveau von mindestens einem im Schritt c) gemessenen Marker höher oder gleich dem Expressionsniveau dieses im Schritt d) gemessenen Markers ist, wenn der Marker ein Marker für die antimikrobielle antioxidative Abwehr oder die DNA, ein Marker für die Hautbarriere, ein Marker für die Adhäsion und die Hautreparatur oder ein vorzugsweise in den Stammzellen exprimierter Marker ist.

2. Verfahren zur Bestimmung einer Formulierung oder eines Wirkstoffs für die Prävention von schädlichen Wirkungen von UV-Strahlung auf die Haut von Kindern von drei Jahren oder jünger, vorzugsweise von zwei Jahren oder jünger, mehr zu bevorzugen von einem Jahr oder jünger, noch mehr zu bevorzugen von sechs Monaten oder jünger und insbesondere von drei Monaten oder jünger, **dadurch gekennzeichnet, dass** das Verfahren die folgenden Schritte umfasst:

    a. Inkontaktbringen einer Kandidatenformulierung oder eines Kandidatenwirkstoffs mit einem Hautmodell, das Keratinozyten umfasst, die von Personen von drei Jahren oder jünger, vorzugsweise von zwei Jahren oder jünger, mehr zu bevorzugen von einem Jahr oder jünger, noch mehr zu bevorzugen von sechs Monaten oder jünger und insbesondere von drei Monaten oder jünger erhalten wurden;
    b. Exponieren des Hautmodells von Schritt a) gegenüber UV-Strahlung;
    c. Messen des Expressionsniveaus einer Kombination von biologischen Markern, die Folgendes umfassen oder aus Folgendem bestehen:

- mindestens einem Marker für Hautentzündung, der aus der Gruppe ausgewählt ist, die aus PTGS2, IL1α, IL8, dem Rezeptor für IL1: IL1-R, dem Antagonisten für den Rezeptor für IL1: IL1-RN, MMP1 a und MMP3 besteht, und
- mindestens einem Marker für die antimikrobielle antioxidative Abwehr oder die DNA, der aus der Gruppe ausgewählt ist, die aus Katalase, Superoxid-Dismutase 1, Defensin β4 und dem Tumorsuppressorprotein p53 besteht; und
- mindestens einem Marker für die Hautbarriere, der aus der Gruppe ausgewählt ist, die aus Filaggrin, Keratin 1, Sciellin, Loricrin, Claudin 1 und Barx2 besteht; und
- mindestens einem Marker für die Adhäsion und die Hautreparatur, der unter dem Zelladhäsionsmolekül 1, dem Molekül CD36 und Fibronektin 1 ausgewählt ist, und
- mindestens einen vorzugsweise in den Stammzellen exprimierten Marker, der unter dem Tumorsuppressorprotein p63 und Survivin ausgewählt ist,

in dem Hautmodell von Schritt b)
d. Vergleichen des Expressionsniveaus von mindestens einem im Schritt c) erhaltenen biologischen Marker mit einem Bezugsexpressionsniveau;
e. Bestimmen, dass die Kandidatenformulierung oder der Kandidatenwirkstoff eine Formulierung oder ein Wirkstoff für die Prävention von schädlichen Wirkungen von UV-Strahlung auf die Haut von Kindern von drei Jahren oder jünger, vorzugsweise von zwei Jahren oder jünger, mehr zu bevorzugen von einem Jahr oder jünger, noch mehr zu bevorzugen von sechs Monaten oder jünger und insbesondere von drei Monaten oder jünger wirksam ist, wenn:

(i) das Expressionsniveau von mindestens einem im Schritt c) gemessenen Marker niedriger oder gleich dem Expressionsniveau dieses im Schritt d) gemessenen Markers ist, wenn der Marker ein Marker für Hautentzündung ist; und
(ii) das Expressionsniveau von mindestens einem im Schritt c) gemessenen Marker höher oder gleich dem Expressionsniveau dieses im Schritt d) gemessenen Markers ist, wenn der Marker ein Marker für die antimikrobielle antioxidative Abwehr oder die DNA, ein Marker für die Hautbarriere, ein Marker für die Adhäsion und die Hautreparatur oder ein vorzugsweise in den Stammzellen exprimierter Marker ist.

**3.** Verfahren nach Anspruch 1 oder 2, **dadurch gekennzeichnet, dass** der Schritt c) die Messung des Expressionsniveaus einer Kombination von biologischen Markern umfasst, die PTGS2, IL1α, IL8, IL1-R, IL1-RN, MMP1, MMP3, Katalase, Superoxid-Dismutase 1, Defensin β4, das Tumorsuppressorprotein p53, Filaggrin, Keratin 1, Sciellin, Loricrin, Claudin 1, Barx2, das Zelladhäsionsmolekül 1, das Molekül CD36, Fibronektin 1, das Tumorsuppressorprotein p63 und Survivin umfasst oder daraus besteht.

## Claims

**1.** A method to evaluate the in vitro efficacy of a formulation or active ingredient in preventing the harmful effects of UV on the skin of children aged three years or younger, preferably two years or younger, more preferably one year or younger, further preferably six months or younger and in particular three months or younger, **characterized in that** said method comprises the following steps:

a. contacting said formulation or said active ingredient with a skin model comprising keratinocytes obtained from subjects aged three years or younger, preferably two years or younger, more preferably one year or younger, further preferably six months or younger and in particular three months or younger;
b. exposing the skin model of step a) to UV;
c. measuring the expression level of a combination of biomarkers comprising or consisting of:

- at least one skin inflammation marker selected from the group consisting of PTGS2, IL1α, IL8, the IL1 receptor: IL1-R, antagonist of the IL1 receptor: IL1-RN, MMP1 a and MMP3, and
- at least one antioxidant antimicrobial defence or DNA marker selected from the group consisting of catalase, superoxide dismutase 1, defensin β4, and the p53 tumour suppressor protein; and
- at least one epidermal barrier marker selected from the group consisting of filaggrin, keratin 1, sciellin, loricrin, claudin 1 and barx2; and
- at least one skin adhesion and repair marker selected from among cell adhesion molecule 1, molecule CD36 and fibronectin 1, and

- at least one marker preferably expressed in stem cells selected from among the p63 tumour suppressor protein and survivin,

in the skin model of step b),
d. comparing the expression level of at least one biomarker obtained at step c) with a reference expression level;
e. determining that said formulation or said active ingredient is efficient in preventing the harmful effects of UV on the skin of children aged three years or younger, preferably two years or younger, more preferably one year or younger, further preferably six months or younger and in particular three months or younger, if:

(i) the expression level of at least one marker measured at step c), is equal to or lower than the expression level of this marker measured at step d), when said marker is a skin inflammation marker; and
(ii) the expression level of at least one marker measured at step c), is equal to or higher than the expression level of this marker measured at step d), when said marker is an antimicrobial antioxidant defence or DNA marker, an epidermal barrier marker, a skin adhesion and repair marker or a marker preferably expressed in stem cells.

**2.** A method for identifying a formulation or active ingredient for the prevention of the harmful effects of UV on the skin of children aged three years or younger, preferably two years or younger, more preferably one year or younger, further preferably six months or younger and in particular three months or younger, **characterized in that** said method comprises the following steps:

a. contacting a candidate formulation or candidate active ingredient with a skin model comprising keratinocytes obtained from subjects aged three years or younger, preferably two years or younger, more preferably one year or younger, further preferably six months or younger and in particular three months or younger;
b. exposing the skin model of step a) to UV;
c. measuring the expression level of a combination of biomarkers comprising or consisting of:

- at least one skin inflammation marker selected from the group consisting of PTGS2, IL1$\alpha$, IL8, the IL1 receptor: IL1-R, antagonist of the IL1 receptor: IL1-RN, MMP1 a and MMP3, and
- at least one antioxidant antimicrobial defence or DNA marker selected from the group consisting of catalase, superoxide dismutase 1, defensin $\beta$4, and the p53 tumour suppressor protein; and
- at least one epidermal barrier marker selected from the group consisting of filaggrin, keratin 1, sciellin, loricrin, claudin 1 and barx2; and
- at least one skin adhesion and repair marker selected from among cell adhesion molecule 1, molecule CD36 and fibronectin 1; and
- at least one marker preferably expressed in stem cells selected from among the p63 tumour suppressor protein and survivin,

in the skin model of step b),
d. comparing the expression level of at least one biomarker obtained at step c) with a reference expression level;
e. determining whether said candidate formulation or said candidate active ingredient is a formulation or active ingredient for the prevention of the harmful effects of UV on the skin of children aged three years or younger, preferably two years or younger, more preferably one year or younger, further preferably six months or younger and in particular three months or younger, if:

(i) the expression level of at least one marker measured at step c), is equal to or lower than the expression level of this marker measured at step d), when said marker is a skin inflammation marker; and
(ii) the expression level of at least one marker measured at step c), is equal to or higher than the expression level of this marker measured at step d), when said marker is an antimicrobial antioxidant defence or DNA marker, an epidermal barrier marker, a skin adhesion and repair marker or a marker preferably expressed in stem cells.

**3.** The method according to claim 1 or 2, **characterized in that** step c) comprises measurement of the expression level of a combination of biomarkers comprising or consisting of PTGS2, IL1$\alpha$, IL8, IL1-R, IL1-RN, MMP1, MMP3, catalase, superoxide dismutase 1, defensin $\beta$4, p53 tumour suppressor protein, filaggrin, keratin 1, sciellin, loricrin, claudin 1, barx2, cell adhesion molecule 1, molecule CD36, fibronectin 1, p63 tumour suppressor protein and survivin.

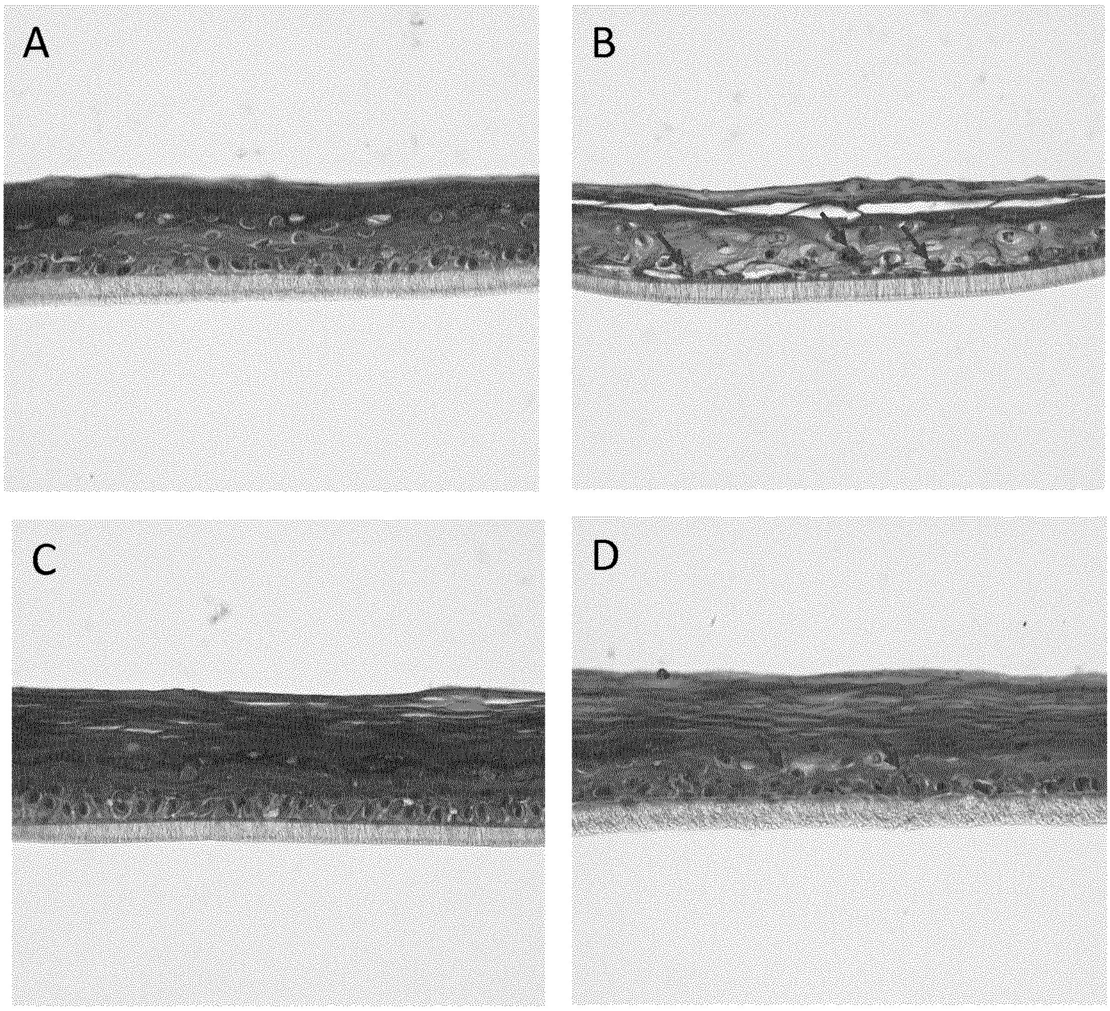


Fig. 1

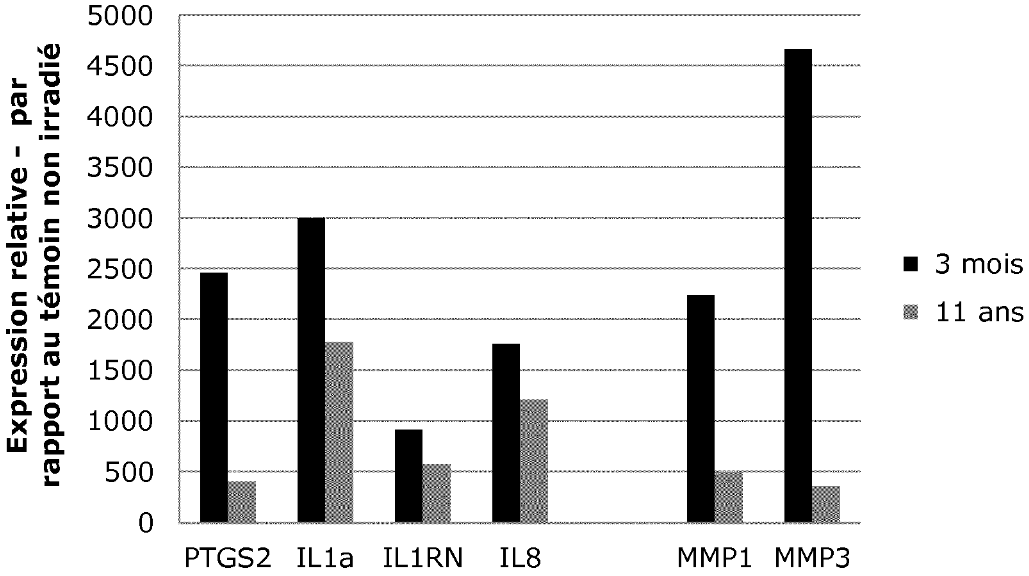
Expression relative - par rapport au témoin non irradié
5000
4500
4000
3500
3000
2500
2000
1500
1000
500
0
PTGS2
IL1a
IL1RN
IL8
MMP1
MMP3
3 mois
11 ans

Fig. 2

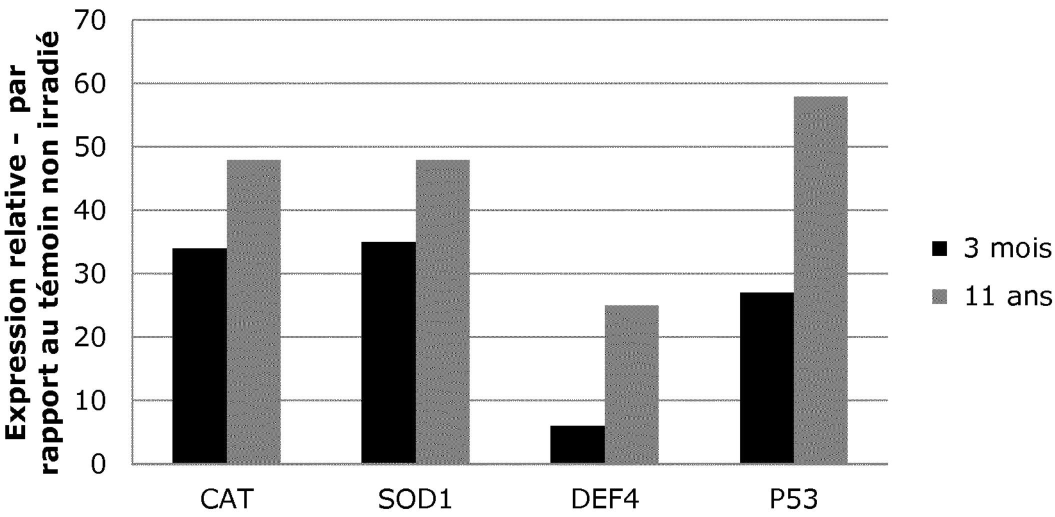
Expression relative - par rapport au témoin non irradié
70
60
50
40
30
20
10
0
CAT
SOD1
DEF4
P53
3 mois
11 ans

Fig. 3

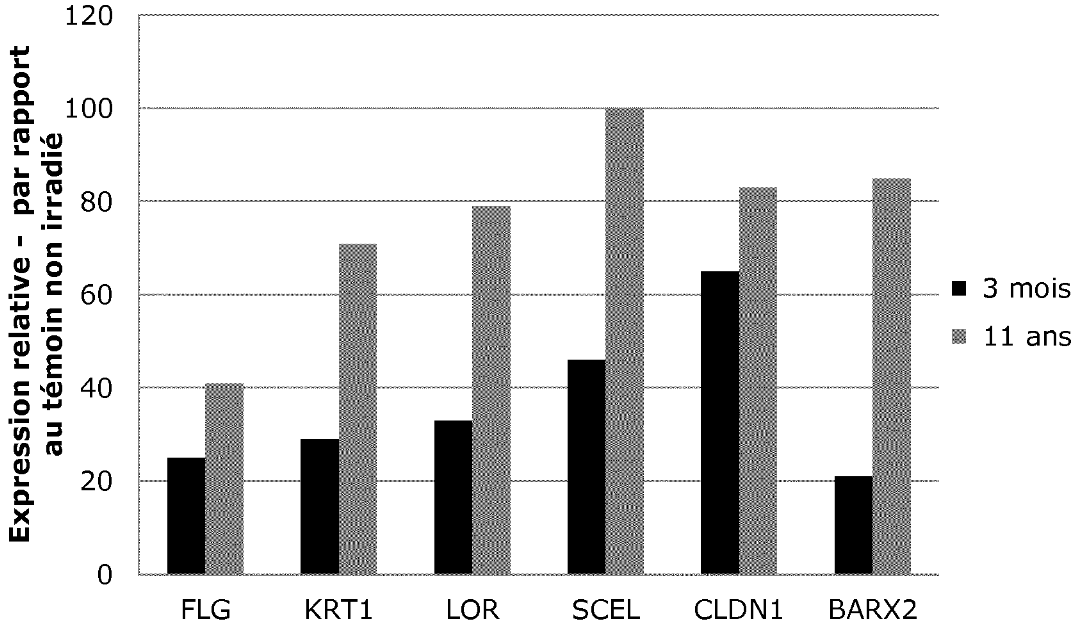
120
100
80
60
40
20
0
Expression relative - par rapport au témoin non irradié
FLG
KRT1
LOR
SCEL
CLDN1
BARX2
3 mois
11 ans

Fig. 4

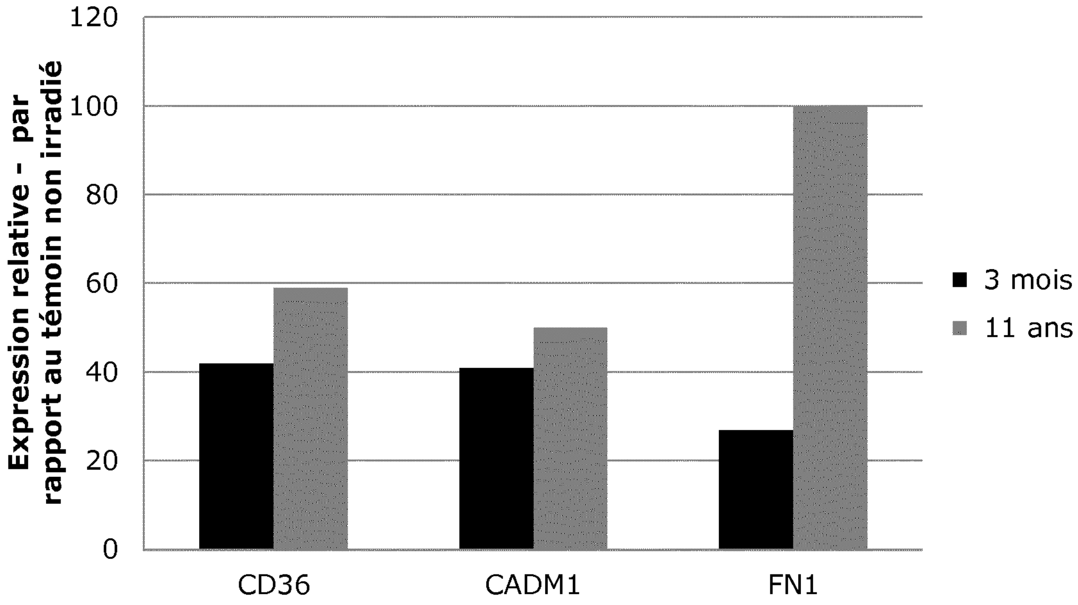
120
100
80
60
40
20
0
Expression relative - par rapport au témoin non irradié
CD36
CADM1
FN1
3 mois
11 ans

Fig. 5

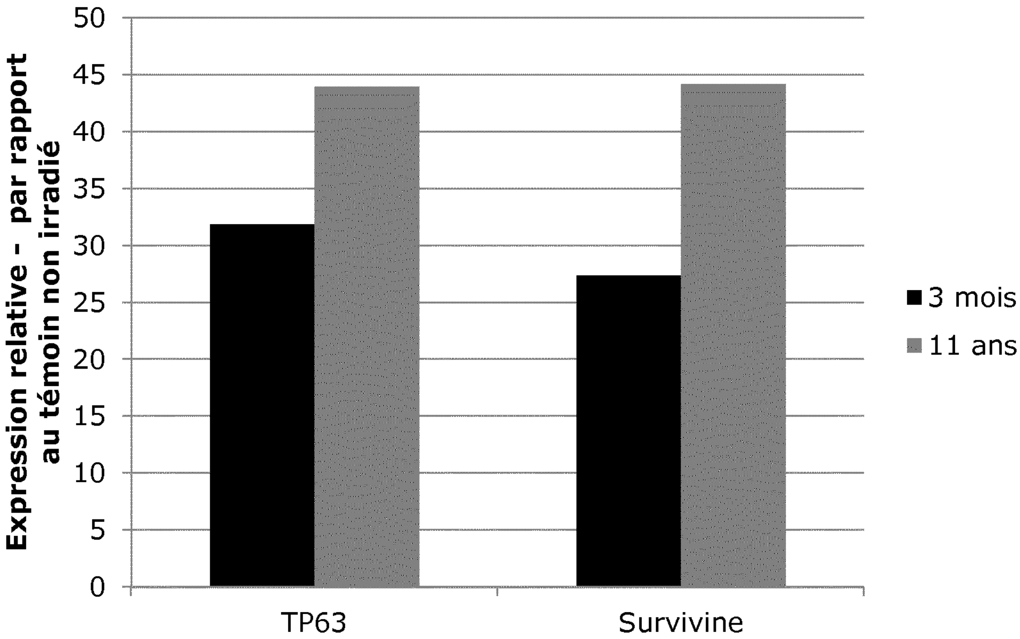
50
45
40
35
30
25
20
15
10
5
0
Expression relative - par rapport au témoin non irradié
TP63
Survivine
3 mois
11 ans

Fig. 6

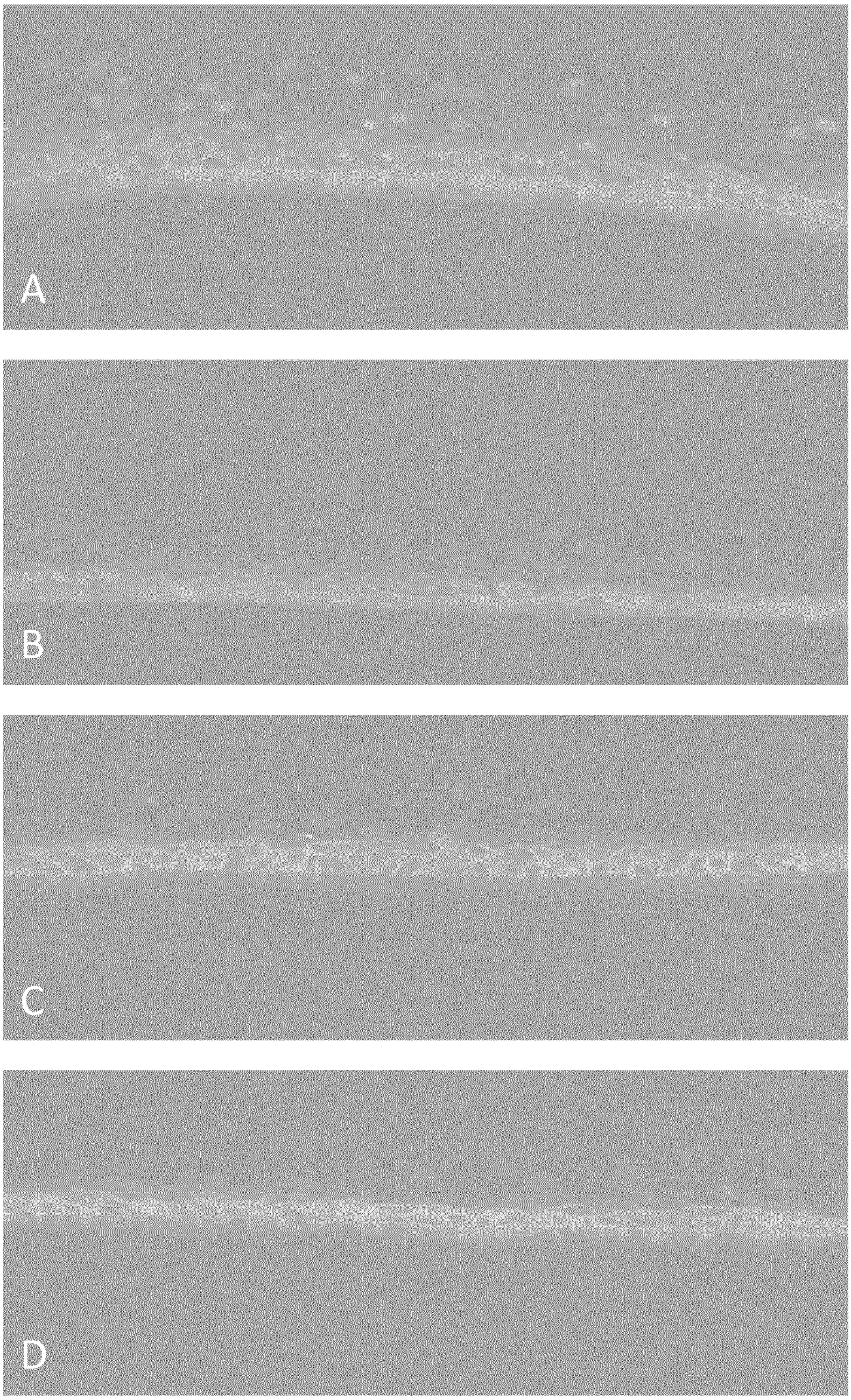


Fig. 7

A

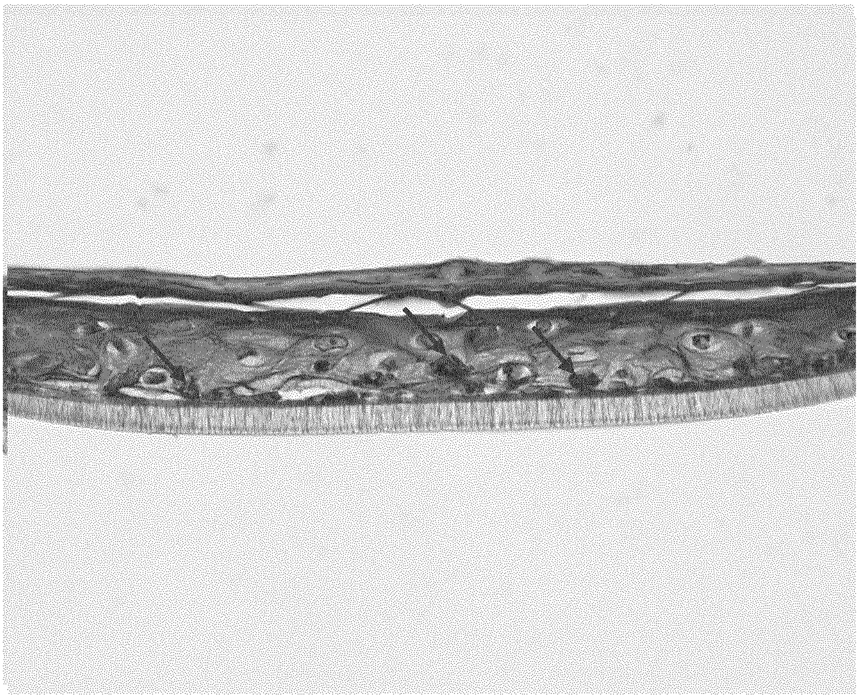

B

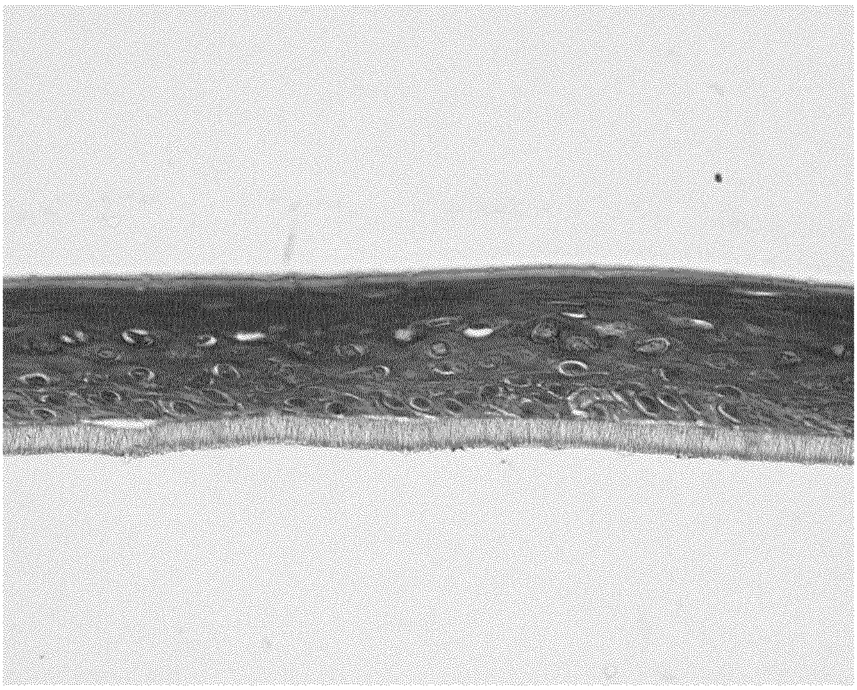

Fig. 8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 2013064926 W
- EP 29678 A
- EP 285471 A
- EP 789074 A
- EP 1451302 B1
- EP 1878790 B1
- EP 1974718 A
- US 20070148771 A
- US 20100099576 A
- WO 02070729 A
- WO 2006063864 A
- WO 2006063865 A
- WO 2007064305 A
- US 4882127 A
- US 4849077 A
- US 7556922 B
- US 6723513 B
- WO 03066896 A
- WO 2007111924 A
- US 20080020392 A
- WO 2006084132 A
- US 20090186349 A
- US 20090181860 A
- US 20090181385 A
- US 20060275782 A
- EP 1141399 B1
- FR 1351136
- WO 2004012496 A
- WO 2004012752 A
- WO 2004016106 A
- WO 2007057439 A
- WO 9847479 A
- WO 200121150 A
- WO 0121605 A
- WO 0151596 A
- WO 2005105123 A
- WO 0062789 A
- WO 2005102259 A
- WO 2008080974 A
- WO 2004112742 A
- WO 2008009709 A
- FR 0955344
- FR 0958525
- FR 0958529
- FR 1262234
- FR 1061055
- FR 1061047
- FR 1061051
- FR 2822821
- FR 2857596
- WO 2004050052 A
- WO 2004050079 A
- WO 2004112741 A

### Littérature non-brevet citée dans la description

- **STANTON WR.** *Health Promot Int.,* 2004, vol. 19 (3), 369-78
- **PALLER AS et al.** *Pediatrics,* 2011, vol. 128, 92-10
- **WOLNICKA-GLUBISZ A ; NOONAN FP.** *Photochem Photobiol Sci.,* 2006, vol. 5, 254
- **LECLERE-BIENFAIT et al.** *J. Invest. Dermatol.,* 2013, S106
- **PEUS et al.** *Free Rad Biol & Med,* 2001, vol. 30 (4), 452-432
- **WU et al.** *Mol Immunol,* 2008, vol. 45 (8), 2288-2296
- **BAUDOUIN et al.** *J. Invest. Dermatol.,* 2013, S106
- **MARCHBANK et al.** *J Derm Sci,* 2003, vol. 32, 71-74
- **LEE et al.** *Brit J Dermatol,* 2005, vol. 152, 52-59
- **LEE et al.** *Photodermatology, Photoimmunology & Photomedecine,* 2005, vol. 21, 45-52
- **ENK et al.** *Oncogene,* 2006, vol. 25, 2601-2614
- **ROSDY et al.** *In Vitro Toxicol.,* 1997, vol. 10 (1), 39-47
- **PONEC et al.** *J Invest Dermatol.,* 1997, vol. 109 (3), 348-355
- **PONEC et al.** *Int J Pharm.,* 2000, vol. 203 (1-2), 211-225
- **SCHMALZ et al.** *Eur J Oral Sci.,* 2000, vol. 108 (5), 442-448
- **BLACK et al.** *Tissue Eng,* 2005, vol. 11 (5-6), 723-733
- **DONGARI-BATGTZOGLOU ; KASHLEVA.** *Nat Protoc,* 2006, vol. 1 (4), 2012-2018
- **BECHTOILLE et al.** *Tissue Eng,* 2007, vol. 13 (11), 2667-2679

- **VRANA et al.** *Invest Ophthalmol Vis Sci,* 2008, vol. 49 (12), 5325-5331
- **KINICOGLU et al.** *Biomaterials,* 2009, vol. 30 (32), 6418-6425
- **AUXENFANS et al.** *Eur J Dermatol,* 2009, vol. 19 (2), 107-113
- **KINICOGLU et al.** *Biomaterials,* 2011, vol. 32 (25), 5756-5764
- **COSTIN et al.** *Altern Lab Anim,* 2011, vol. 39 (4), 317-337
- **AUXENFANS et al.** *J Tissue Eng Regen Med,* 2012, vol. 6 (7), 512-518
- **LEQUEUX et al.** *Skin Pharmacol Physiol,* 2012, vol. 25 (1), 47-55
- **LEIGH et al.** *Arch Dermatol Res.,* 1994, vol. 286 (1), 53-61
- **DIFFEY BL et al.** *J Am Acad Dermatol,* 2000, vol. 43 (6), 1024-35
- **VAHLQUIST.** *Acta Derm Venereol,* 2000, vol. 80, 161
- **SHENDURE ; JI.** *Nat Biotechnol.,* 2008, vol. 26 (10), 1135-45
- **PIHLAK et al.** *Nat Biotechnol.,* 2008, vol. 26 (6), 676-684
- **FULLER et al.** *Nature Biotechnol.,* 2009, vol. 27 (11), 1013-1023
- **MARDIS.** *Genome Med.,* 2009, vol. 1 (4), 40
- **METZKER.** *Nature Rev. Genet.,* 2010, vol. 11 (1), 31-46
- **EISENBERG et al.** *Trends in Genetics,* 2003, vol. 19, 362-365
- **POUMAY et al.** *Arch Dermatol Res,* 2004, vol. 296, 203-11